# EUROPEAN PATENT APPLICATION

(11) **EP 4 044 195 A1**
(43) Date of publication of application: **17.08.2022**
(21) Application number: 19946399.3
(22) Date of filing: 25.09.2019
(51) Int. Cl.: G16H 70/20

(54) **INFUSION PUMP AND INFUSION PARAMETER SETTING METHOD**

(71) Applicant: Shenzhen Mindray Scientific Co., Ltd., Shenzhen, Guangdong 518000 (CN)
(72) Inventor: PAN, Ruiling, Shenzhen, Guangdong 518000 (CN); ZOU, Xiaoling, Shenzhen, Guangdong 518000 (CN); JIANG, Xia, Shenzhen, Guangdong 518000 (CN); LI, Youlan, Shenzhen, Guangdong 518000 (CN); XU, Zhen, Shenzhen, Guangdong 518000 (CN)
(74) Representative: KIPA AB
(86) International application number: PCT/CN2019/107862
(87) International publication number: WO 2021/056267

(57) **Abstract**

An infusion pump (100) and an infusion parameter setting method therefor. The method includes: displaying an image (1002) of at least one infusion parameter setting item on a display system (160), where the infusion parameter setting item image is operable to present infusion parameter content, an image (1002) of the infusion parameter setting item triggered by a user and an image (1006) of an editing item corresponding to the triggered infusion parameter setting item being able to be displayed together on the display system (160) by means of a trigger operation of the user; and when the user performs a trigger operation on the image (1006) of the editing item, content updating being able to be performed on the image (1002) of the infusion parameter setting item on the same screen, without switching between upper-level and lower-level display interfaces. A user can verify edited information in real time and can discover a setting error in a timely manner, such that the infusion safety of a patient is facilitated, and in particular, an infusion parameter can be set more quickly and more accurately, when the life of the patient is in danger.

## Description

### TECHNICAL FIELD

The present disclosure relates to the technical field of medical technologies, and in particular to an infusion pump and an infusion parameter setting method.

### BACKGROUND

In clinical practice, a doctor needs to give medical advice to a patient according to their medical condition. After a nurse gets the medical advice, they will get and dispense drugs, and then connect the dispensed fluid substance (such as a medicinal solution) to an infusion pump by means of an infusion set. In addition, the nurse sets infusion parameters on the infusion pump according to the medical advice, so that the infusion pump performs infusion for the patient according to the set parameters. However, when setting infusion parameters, medical care personnel often need to switch between multiple interfaces of higher and lower levels, for example, jumping from a reading interface to an editing interface for editing, and then jumping back to the reading interface to read the set infusion parameter information. Moreover, most of the current infusion pumps use physical keys for parameter setting, which results in a poor user experience and also makes the entire infusion parameter setting process cumbersome. It is impossible to check the editing information in real time, and it is not conducive to the safety of patient infusion.

### SUMMARY

The present disclosure provides an infusion pump and an infusion parameter setting method, so that an infusion parameter setting process is more convenient, and editing information can be checked in real time, which is beneficial to the safety of patient infusion.

A first aspect of an embodiment of the present disclosure provides an infusion parameter setting method, the method including:
displaying an image of at least one infusion parameter setting item on a display system including at least one display screen, where the infusion parameter setting item image is operable to present infusion parameter content, and the at least one infusion parameter setting item includes a first infusion parameter setting item;
displaying the image of the first infusion parameter setting item and an image of a first editing item corresponding to the first infusion parameter setting item on the display system, when detecting a first trigger event generated based on the first infusion parameter setting item, where the first editing item image is operable to present a content element of the first infusion parameter setting item; and
updating the infusion parameter content in the image of the first infusion parameter setting item based on a content element corresponding to a second trigger event, when detecting the second trigger event generated based on the first editing item.

A second aspect of an embodiment of the present disclosure provides an infusion pump, the infusion pump is used together with an infusion set, and the infusion set is operable to deliver a fluid substance in the infusion set into a patient's body; and the infusion pump includes:
a drive apparatus, which is operable to apply pressure to the infusion set, such that the fluid substance in the infusion set moves directionally;
an input/output system, which is operable to provide an interface between an input/output peripheral and a peripheral device interface;
the input/output peripheral, which includes a display system for providing a visual display interface, where the display system includes at least one display screen; and
a processor, configured to display an image of at least one infusion parameter setting item on the display system, where the infusion parameter setting item image is operable to present infusion parameter content, and the at least one infusion parameter setting item includes a first infusion parameter setting item; and further configured to display the image of the first infusion parameter setting item and an image of a first editing item corresponding to the first infusion parameter setting item on the display system, when detecting a first trigger event generated based on the first infusion parameter setting item, where the first editing item image is operable to present a content element of the first infusion parameter setting item; and update the infusion parameter content in the image of the first infusion parameter setting item based on a content element corresponding to a second trigger event, when detecting the second trigger event generated based on the first editing item.

A third aspect of an embodiment of the present disclosure provides an infusion pump, the infusion pump is used together with an infusion set, and the infusion set is operable to deliver a fluid substance in the infusion set into a patient's body; and the infusion pump includes:
a pump body;
a drive apparatus, which is operable to apply pressure to the infusion set, such that the fluid substance in the infusion set moves directionally;
a pump door, which is movably mounted to the pump body and configured to cover an accommodating cavity for mounting the infusion set, or configured to expose the accommodating cavity for mounting the infusion set, where the pump door has a front that faces outside;
an input/output system, which is operable to provide an interface between an input/output peripheral and a peripheral device interface;
the input/output peripheral, which comprises a display system for providing a visual display interface, where the display system includes at least one display screen; and the display system is disposed on the pump door, the display system extends from the left of a midline of the front of the pump door to the right of the midline of the front of the pump door, and a width of the display system is greater than a height of the display system; and
a processor, configured to, display an image of at least one infusion parameter setting item on the display system, after the pump door is closed onto the pump body, where the infusion parameter setting item image is operable to present infusion parameter content, and the at least one infusion parameter setting item includes a first infusion parameter setting item; and further configured to display the image of the first infusion parameter setting item and an image of a first editing item corresponding to the first infusion parameter setting item on the display system, when detecting a first trigger event generated based on the first infusion parameter setting item, where the first editing item image is operable to present a content element of the first infusion parameter setting item; and update the infusion parameter content in the image of the first infusion parameter setting item based on a content element corresponding to a second trigger event, when detecting the second trigger event generated based on the first editing item.

According to the embodiments of the present disclosure, by means of a user's trigger operation, an image of an infusion parameter setting item triggered by the user and an image of an editing item corresponding to the triggered infusion parameter setting item can be displayed together on the display system; and when the user performs a trigger operation on the image of the editing item, content updating is able to be performed on the image of the infusion parameter setting item on the same screen, without switching between upper-level and lower-level display interfaces. A user can verify edited information in real time, and can discover a setting error in a timely manner, such that the infusion safety of a patient is facilitated, and in particular, an infusion parameter can be set more quickly and more accurately, when the life of the patient is in danger.

### BRIEF DESCRIPTION OF THE DRAWINGS

To more clearly describe the technical solutions in the implementations of the present disclosure, the accompanying drawings required for describing the implementations will be briefly described below. Apparently, the accompanying drawings in the following description show only some of the implementations of the present disclosure, and persons of ordinary skill in the art may still derive other drawings from these accompanying drawings without creative efforts.
FIG. 1 is a hardware structure block diagram of an infusion pump in an embodiment of the present disclosure;
FIG. 2 is a schematic diagram of a peristaltic pressing mechanism of a transfusion pump in an embodiment of the present disclosure;
FIG. 3 is a schematic diagram of a drive mechanism of a syringe pump in an embodiment of the present disclosure;
FIG. 4A is a diagram of an external structure of a transfusion pump in an embodiment of the present disclosure;
FIG. 4B is a diagram of an external structure of a syringe pump in an embodiment of the present disclosure;
FIG. 4C is another diagram of an external structure of a syringe pump in an embodiment of the present disclosure;
FIG. 5Ais a schematic display diagram in an embodiment of the present disclosure;
FIG. 5B is another schematic display diagram in an embodiment of the present disclosure;
FIG. 5C is another schematic display diagram in an embodiment of the present disclosure;
FIG. 5D is another schematic display diagram in an embodiment of the present disclosure;
FIG. 5E is another schematic display diagram in an embodiment of the present disclosure;
FIG. 5F is another schematic display diagram in an embodiment of the present disclosure;
FIG. 6 is a flowchart in an embodiment of the present disclosure;
FIG. 7 is another flowchart in an embodiment of the present disclosure;
FIG. 8Ais a schematic diagram of a display interface change in an embodiment of the present disclosure;
FIG. 8B is another schematic diagram of a display interface change in an embodiment of the present disclosure;
FIG. 8C is another schematic diagram of a display interface change in an embodiment of the present disclosure;
FIG. 8D is another schematic diagram of a display interface change in an embodiment of the present disclosure;
FIG. 9Ais a schematic diagram of a display interface change generated based on a fourth trigger event in an embodiment of the present disclosure;
FIG. 9B is another schematic diagram of a display interface change generated based on a fourth trigger event in an embodiment of the present disclosure;
FIG. 10 is another schematic diagram of a display interface change in an embodiment of the present disclosure;
FIG. 11A is a schematic display diagram related to an infusion mode setting item in an embodiment of the present disclosure; and
FIG. 11B is another schematic display diagram related to an infusion mode setting item in an embodiment of the present disclosure.

### DESCRIPTION OF THE PREFERRED EMBODIMENTS

Reference will now be made in detail to embodiments, and examples of these implementations are illustrated in the accompanying drawings. In the following detailed description, many specific details are illustrated to provide a full understanding of various embodiments described. However, persons of ordinary skill in the art should understand that the various embodiments described can be implemented without these specific details. In other embodiments, well-known methods, processes, components, circuits, and networks are not described in detail, so as not to unnecessarily obscure the embodiments.

It is to be additionally understood that although the terms "first", "second", etc. are used herein in some cases to describe various elements or other objects, these elements or objects should not be limited by these terms. These terms are used merely to distinguish one element/object from another.

The terms used in the description of the various implementations herein are merely for the purpose of describing particular implementations, and are not intended to be limiting. As used in the description of the various embodiments and the appended claims, the singular forms of "a/an" and "the/said" are also intended to include plural forms, unless the context clearly indicates otherwise. It is to be additionally understood that the term "and/or" used herein refers to and covers any or all possible combinations of one or more associated listed items. It is to be additionally understood that the term "include/comprise" used in the specification refers to the presence of the described features, steps, operations, elements, and/or components, but does not exclude the presence or addition of one or more other features, steps, operations, elements, and/or components.

As used herein, depending on the context, the term "if' can be interpreted as "when", "in response to determining", "in response to detecting", or the like. Similarly, depending on the context, the phrases "if it is determined that..." or "if [the stated condition or event] is detected" can be interpreted as "when determining...", "in response to determining...", "when detecting [the stated condition or event]", or "in response to detecting [the stated condition or event]".

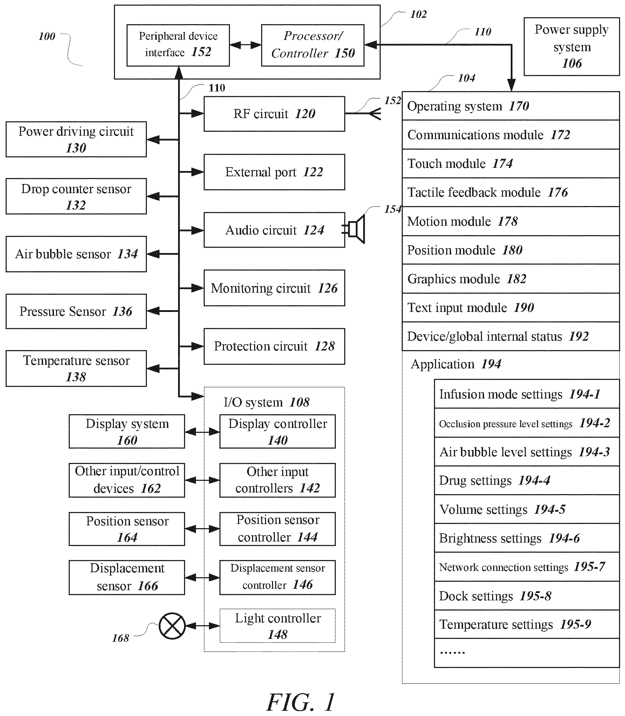

FIG. 1 is a hardware structure block diagram of an infusion pump in some embodiments of the present disclosure; The infusion pump 100 includes a control platform 102, a memory 104, a power supply system 106, an input/output (I/O) system 108, an RF circuit 120, an external port 122, an audio circuit 124, a monitoring circuit 126, a protection circuit 128, a power driving circuit 130, a drop counter sensor 132, an air bubble sensor 134, a pressure sensor 136, and a temperature sensor 138. These components communicate with each other via one or more communications buses or signal lines 110. The control platform 102 includes a processor 150 and a peripheral device interface 152.

The infusion pump 100 may be any medical device, including but not limited to a transfusion pump, a syringe pump, etc., that performs an infusion operation set by a user based on a fluid substance dispensed by the user, and controllably infuses the configured fluid substance (such as a medicinal solution) into a patient's body. The infusion pump 100 can be used together with an infusion set (e.g., an infusion tube or a syringe). It should be understood that the infusion pump 100 is merely an example, and the components thereof may be more or fewer than the components shown, or may have a different component configuration. The various components described in combination with FIG. 1 may be implemented by hardware, software, or a combination of software and hardware, including one or more signal processing and/or application-specific integrated circuits.

The memory 102 may include a high-speed random access memory, and may further include a non-volatile memory, for example, one or more magnetic disk storage devices, flash memory devices, or other non-volatile solid-state storage devices. In some embodiments, the memory 104 may further include a memory remote from the one or more processors 150, for example, a network attached memory accessed via the RF circuit 120 or the external port 122 and a communications network (not shown). The communications network may be the Internet, one or more intranets, a local area network (LAN), a wide area network (WLAN), a storage area network (SAN), etc., or an appropriate combination thereof. The processor 150 can control access to the memory 104 by other components of the infusion pump 100 than the peripheral device interface 152.

The peripheral device interface 152 couples input and output peripherals of the infusion pump 100 to the processor 150 and the memory 104. The one or more processors 150 run various software programs and/or instruction sets stored on the memory 104, to perform various functions of the infusion pump 100 and to process data.

In some embodiments, the peripheral device interface 152 and the processor 150 may be implemented on a single chip. In some embodiments, they may be implemented on a plurality of discrete chips.

The RF (radio frequency) circuit 120 receives and transmits electromagnetic waves. The RF circuit 120 converts an electrical signal into an electromagnetic wave, or converts an electromagnetic wave into an electrical signal, and communicates with a communications network and another communications device via electromagnetic waves. The RF circuit 112 may include well-known circuits for performing these functions, including but not limited to, an antenna system, an RF transceiver, one or more amplifiers, a tuner, one or more oscillators, a digital signal processor, a CODEC chipset, a subscriber identity module (SIM) card, a memory, etc. The RF circuit 120 may communicate with a network and another device through wireless communication. The network may be the World Wide Web (WWW), an intranet and/or a wireless network such as a cellular telephone network, or a wireless local area network (LAN) and/or a metropolitan area network (MAN). The wireless communications may use any one of a variety of communications standards, protocols, and technologies, including but not limited to, Global System for Mobile Communications (GSM), Enhanced Data GSM Environment (EDGE), Wideband Code Division Multiple Access (WCDMA), Code Division Multiple Access (CDMA), Time Division Multiple Access (TDMA), Bluetooth (e.g., IEEE 802.15.1), Wireless Fidelity (Wi-Fi) (e.g., IEEE 802.11a, IEEE 802.11b, IEEE 802.11g, and/or IEEE 802.11n), Voice over Internet Protocol (VoIP), Wi-MAX, protocols for email, instant messaging, and/or short message service (SMS), or any other suitable communications protocol, including those communications protocols not yet developed as of the filing date of this specification.

The external port 122 provides a wired communications interface between the infusion pump 100, another device (e.g., a mounting dock, a central station, or a monitor), or a user (a computer or another communications device). In some embodiments, the wired communications interface may be a communications interface controlled by a CAN bus protocol, a communications interface controlled by a serial communications protocol (e.g., RS485 or RS232), or a universal serial bus (USB). The external port 122 is suitable to be coupled directly or indirectly via a network (e.g., the Internet or a LAN) to another device or user.

The audio circuit 124 and a speaker 154 provide an audio interface between the user and the infusion pump 100. The audio circuit 124 receives audio data from the peripheral device interface 152, converts the audio data to an electrical signal, and transmits the electrical signal to the speaker 154. The speaker 154 converts the electrical signal into a human-perceivable acoustic wave.

The monitoring circuit 126 may include a fault detection circuit for prompting a status of the one or more processors 150.

The protection circuit 128 may include a hardware protection apparatus (e.g., a fuse or a TVS diode) to protect the electrical safety of the various components in the infusion pump 100. The processor 150 drives a power device (not shown in the figure) of the infusion pump 100 by means of the power driving circuit 130, so that the power device can move in a controlled manner under the driving of the processor 150, and during the movement, one or more force transmission/conversion devices (such as a gear, a drive shaft, a screw, a nut, or a slider) drive a control object (such as a pump door, an infusion line clamp, a peristaltic pressing mechanism, or a push-pull box) to move. The power device may be an electromagnetic apparatus that achieves electric energy conversion or transfer according to the law of electromagnetic induction, such as a permanent magnet (PM) motor, a reactive (VR) motor, and a hybrid (HB) motor. In some embodiments, driven by the processor 150, the motor drives the control object (such as the pump door, the infusion line clamp, a pump blade, or the push-pull box) of the infusion pump 100 to move, so that the control object achieves a preset motion state.

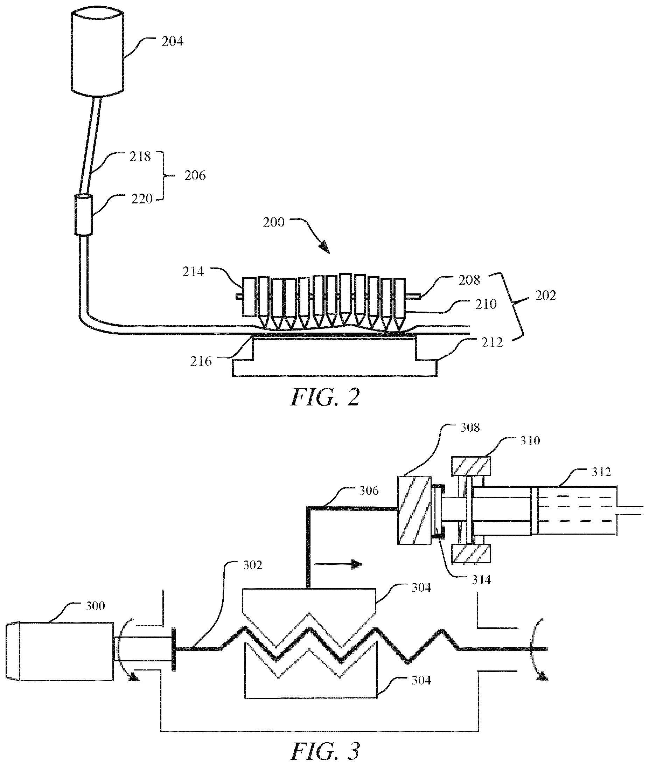

In some embodiments, as shown in FIGS. 1 and 2, the peristaltic pressing mechanism 200 includes a camshaft 208, a pump blade set 210, and a pressing plate 212. The processor 150 in the infusion pump 100 issues instructions such as a rotational speed or a position, and the power driving circuit 130 drives the power device 214 (such as a motor) to operate according to the specified rotational speed and direction. During the rotation process, the power device 214 drives the connected camshaft 208 to rotate. During the rotation of the camshaft 208, the pump blade set 210 on the camshaft 208 performs linear reciprocating motion, that is, pump blades in the pump blade set 210 perform linear reciprocating motion in sequence. The pump blade set 210 cooperates with the pressing plate 212 to press and release an outer wall of the infusion tube 218 in sequential reciprocation, to drive the liquid in the infusion tube 218 to flow continuously and directionally. A deceleration mechanism may be further provided between the power device 214 and the camshaft 208, to ensure that the rotational speed of the pump blade set 210 is stable and uniform.

In some embodiments, as shown in FIGS. 1 and 3, the infusion set is a syringe 312. The push-pull box 308 is configured to clamp a plunger 314 of the syringe 312. The processor 150 in the syringe pump 100 issues instructions such as a rotational speed or a position, and the power driving circuit 130 drives the power device (such as a motor 300). The motor 300 drives a screw 302 and a nut 304 by means of a deceleration mechanism, to convert the rotational motion of the motor 300 into the linear motion of the nut 304. The nut 304 is connected to a push rod 306 matching the syringe 312, and the push rod 306 is connected to the push-pull box 308. The push-pull box 308 can push the plunger 314 matching the syringe 312 for injection and infusion. Setting the rotational speed of the motor 300 can adjust a speed of pushing the matching syringe 312 by the motor, so as to adjust the given infusion dose and infusion rate.

In some embodiments, the drop counter sensor 132 may be used together with a drip chamber 220 of the infusion set 206, to measure a flow rate or flow of drops in the drip chamber 220.

In some embodiments, one or more air bubble sensors 134 are configured to detect the presence and size of air bubbles in the infusion set. The air bubble sensor 134 may be an ultrasonic sensor or an infrared sensor or the like.

In some embodiments, the pressure sensor 136 can respond to a pressure value of a detected object, convert the pressure value into an electrical signal for detection, and send the electrical signal to the control platform 102. The pressure sensor may be a resistive strain gauge pressure sensor, a semiconductor strain gauge pressure sensor, a piezoresistive pressure sensor, an inductive pressure sensor, a capacitive pressure sensor, a resonant pressure sensor, a fiber-optic pressure sensor, or a capacitive acceleration sensor. In some embodiments, the pressure sensor 136 may be configured to measure an internal pressure of the infusion set or an external pressure of the infusion set. In some embodiments, the pressure sensor 136 may be further configured to detect a service status of the detected object (such as the infusion tube 218 or the syringe 312). In some embodiments, the pressure sensor 136 may detect occlusion in the infusion set, or detect whether the infusion set leaks.

In some embodiments, the infusion pump 100 has a heating device for heating the liquid in the infusion set, and the temperature sensor 138 may be configured to measure a real-time temperature of the liquid. At the same time, the temperature sensor converts the temperature value into an electrical signal for detection, and sends the electrical signal to the control platform 102. The control platform 102 can display the real-time temperature by means of the display system 160, and can also control to turn on/off the heating device based on the temperature value.

The input/output (I/O) system 108 provides an interface between an input/output peripheral and a peripheral device interface 152 of the infusion pump 100. The input/output peripheral may be the display system 160, a position sensor 164, a displacement sensor 166, a light assembly 168, and other input/control devices 162. The I/O system 108 may include a display controller 140, a position sensor controller 144, a displacement sensor controller 146, a light controller 148, and one or more input controllers 142. One or more controllers in the I/O system 108 receive/transmit electrical signals from/to the input/output peripheral. The one or more input controllers 142 receive/transmit electrical signals from/to the other input/control devices 162. The other input/control devices 162 may include a physical button (e.g., a push button, a rocker button, or a touch button), a slider switch, a joystick, and the like. In some embodiments, the other input/control devices 162 may include a physical button for an emergency stop of infusion.

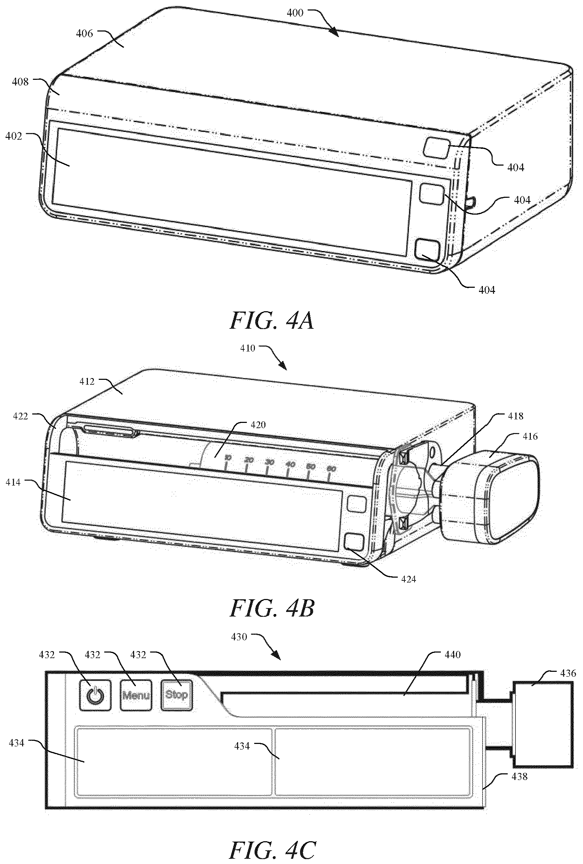

In some embodiments, as shown in FIGS. 1, 4A, 4B, and 4C, the display system 160 may include at least one display screen, such as a display screen 402 in FIG. 4A, a display screen 414 in FIG. 4B, or display screens 434 in FIG. 4C. The display screen provides an output interface between a transfusion pump 400/a syringe pump 410/a syringe pump 430 and a user, which provides a visual display interface. In some embodiments, the display screen 402/414/434 may include a touch layer and a display layer that are stacked. The touch layer provides an input/output interface between the transfusion pump 400/the syringe pump 410/the syringe pump 430 and the user. The touch layer may include a resistive screen, a surface acoustic wave screen, an infrared touchscreen, an optical touchscreen, a capacitive screen, a nano-film, or the like, and is an inductive display device that can receive an input signal such as a contact. A visual output optionally includes graphics, text, charts, video, and a combination thereof. Some or all of the visual outputs may correspond to user interface objects, more details of which are described herein.

The display screen may also receive the user's input based on the sense of touch and/or contact. The touch layer of the display screen forms a touch-sensitive surface that receives the user's input. The touch layer and the display controller 140 (along with any associated modules and/or instruction sets in the memory 104) detect a contact on the touch layer (and any movement or interruption of the contact), and convert the detected contact into interaction with user interface objects such as one or more virtual keys displayed on the touch layer. In an exemplary embodiment, a point of contact between the touch layer and the user corresponds to one or more fingers of the user. The touch layer may use a liquid crystal display (LCD) technology or a light-emitting polymer display (LPD) technology, although other display technologies may be used in other embodiments. The touch layer and the display controller 140 may detect a contact and the movement or interruption of the contact using any of a variety of touch-sensitive technologies, including but not limited to, capacitive, resistive, infrared, and surface acoustic wave technologies, as well as other proximity sensor arrays, or other technologies for determining one or more points in contact with the touch layer.

The position sensor 164 may sense a position of the detected object, convert the position into an electrical signal for detection, and send the electrical signal to the control platform 102 through the I/O system 108. The position sensor may be a contact sensor that generates a signal due to the contact and pressing between two objects, such as a travel switch, a two-dimensional matrix position sensor; or may be a proximity sensor that generates a signal when the proximity of two objects reaches a preset distance, such as electromagnetic, photoelectric, differential transformer, eddy current, capacitive, reed switch, ultrasonic, or Hall-type. The detected object may include an infusion set, a pump door, a pump blade, an infusion line clamp, a push rod, etc. In some embodiments, a position of the pump door may be detected using a Hall-type position sensor. In some embodiments, a position of the pump blade may be detected using a photoelectric position sensor. In some embodiments, whether the infusion set is disposed at a preset position may be detected using a photoelectric position sensor. In some embodiments, a position status of a gripping mechanism of the syringe may be detected using a photoelectric position sensor. In some embodiments, a clamping position of the infusion line clamp may be detected using a photoelectric position sensor.

The displacement sensor 166 can respond to a position change of the detected object relative to a reference position, convert the position change into an electrical signal for detection, and send the electrical signal to the control platform 102 through the I/O system 108. The displacement sensor 106 may be inductive, capacitive, ultrasonic, or Hall-type. In some embodiments, a position change of the pump door may be monitored using a potentiometer. In some embodiments, a position change of a slider of the syringe pump may be monitored using a potentiometer. In some embodiments, a change in an outer diameter of the infusion set (e.g., the syringe) may be monitored using a rotary potentiometer.

The light assembly 168 may include a visual alarm element for prompting that the infusion pump 100 is in an anomalous state. The light assembly 168 responds to the driving of the processor 150 alone; or the light assembly 168 may correspondingly cooperate with the speaker 154 to respond to the driving of the processor 150, for example, the light changes in color or brightness according to the tone and frequency of the alarm sound. The light assembly 168 may include indicator lights of components such as the power supply and the CPU, or an infusion fault status warning light. The light assembly 168 may also include a visual lighting element for facilitating viewing a status of the structure or components of the infusion pump 100 when ambient light is poor.

The infusion pump 100 further includes a power supply system 106 for supplying power to various components. The power supply system 106 may include a power management system, one or more power supplies (e.g., a battery or an alternating current (AC)), a charging system, a power failure detection circuit, a power converter or inverter, a power status indicator (e.g., a light-emitting diode (LED)), and may also include any other components associated with power generation, management, and distribution.

In some embodiments, a software component includes an operating system 170, a communications module (or instruction set) 172, a touch module (or instruction set) 174, a tactile feedback module (or instruction set) 176, a motion module (or instruction set) 178, a position module (or instruction set) 180, a graphics module (or instruction set) 182, a text input module (or instruction set) 190, a device/global internal status (or instruction set) 192, and one or more applications (or instruction set) 194.

The operating system 170 (e.g., an embedded operating system such as Darwin, RTXC, LINUX, UNIX, OS, or WINDOWS) includes various software components and/or drivers for controlling and managing routine system tasks (e.g., memory management, storage device control, or power management) and facilitating communication between various hardware and software components.

The communications module 172 facilitates communication with other devices via one or more external ports 122, and further includes various software components for processing data received by the RF circuit 120 and/or the external port 122.

In some embodiments, the touch module 174 may selectively detect contacts with the display system 160 or other touch-sensitive devices (e.g., a touch button and a touch pad). For example, the touch module 174 detects contact with the display system 160 together with the display controller 140. The touch module 174 includes various software components for performing various operations associated with detection of a contact with the display system 160 (which may be implemented using a finger or a stylus, etc.). For example, the operations include determining the presence of the contact (e.g., detecting a finger-down time), determining a strength of the contact (such as a force or pressure of the contact), determining whether the contact moves (e.g., detecting one or more finger drag events), and tracking the movement on the display screen and determining whether the contact stops (e.g., detecting a finger-up time or a contact break). The operation of determining the movement of a contact point may include determining a rate (amplitude), a velocity (amplitude and direction), and/or an acceleration (including the amplitude and/or direction) of the contact point. These operations can be applied to a single-point contact or a multi-point contact. In some embodiments, the touch module 174 detects contact with the other touch devices together with the display controller 140.

The touch module 174 may be configured to detect the user's gesture input. Different gestures of the user on the touch-sensitive device have different contact patterns (e.g., a combination of one or more of the detected position, time, or strength of contact). For example, detecting a single-finger tap gesture includes detecting a finger-down event, and then detecting a finger-up event at or near the same position as the finger-down event. For example, detecting a finger swipe gesture on the surface of a touch device includes detecting a finger-down event, then monitoring one or more finger drag events, and then detecting a finger-up event. Similarly, tap, swipe, drag, and other gestures of the stylus are optionally detected by detecting specific contact patterns of the stylus.

The tactile feedback module 176 includes various software components for generating instructions, so as to use one or more tactile output generators (not shown in the figure) to generate a tactile output at one or more positions of the infusion pump 100 in response to the interaction between the user and the infusion pump 100. For example, upon the detection of contact on the surface of the touch device, the color of the graphics or text on the touch device changes, or sound or vibration is issued.

The position module 180 includes software components for performing various operations related to detecting a device position and detecting changes in the device position.

The graphics module 182 includes various known software components for rendering or displaying graphics on the display system 160 or a display screen of another external device, including components for changing the visual impact of displayed graphics (e.g., brightness, transparency, saturation, contrast, or other visual properties). In the embodiments herein, the term "graphics" includes any object that can be displayed to a user, including, without limitation, text, web pages, icons (such as user interface objects of software), digital images, videos, animations, or the like. In some embodiments, the graphics module 182 stores data representing graphics to be used. Each graphic may be assigned a corresponding code. The graphics module 182 receives, from an application or the like, one or more codes for specifying graphics to be displayed, if necessary, along with coordinate data and other graphics attribute data, and then generates and outputs screen image data to the display controller 140.

The text input module 190 provides various software components for entering text in one or more applications. Specifically, the text input module can be used to enter various infusion parameters, including drug name, infusion rate, alarm threshold, etc.

In some embodiments, the memory 104 stores the device/global internal status 192. The device/global internal status 157 includes one or more of the following: an active application status, indicating which applications, if any, are currently active; a display status, indicating what applications, views, or other information occupy various regions of the display system 160; a sensor status, including information obtained from the device's various sensors and other input or control devices 162; and position and/or orientation information about a position and/or posture of the device.

In some embodiments, the memory 104 (in FIG. 1) stores at least one application 194. The application 194 may include infusion mode 194-1, occlusion pressure level settings 194-2, air bubble level settings 194-3, drug settings 194-4, volume settings 194-5, brightness settings 194-6, network connection settings 195-7, dock settings 195-8, or temperature settings 195-9. The infusion mode settings 194-1 may include a combination of preset infusion parameters, to adapt to requirements of different use scenarios. The occlusion pressure level settings 194-2 may include providing an interface for the user to input different occlusion pressure levels, and an occlusion alarm threshold of the infusion pump 100 can be adjusted by inputting different occlusion pressures, to adapt to requirements of different use scenarios. The air bubble level settings 194-3 may include providing an interface for the user to input different air bubble levels, and an air bubble alarm threshold of the infusion pump 100 can be adjusted by inputting different air bubble levels, to adapt to requirements of different use scenarios. The drug settings 194-4 may include providing an interface or the like for the user to input different drug names, drug abbreviations, and/or drug colors, and pre-infusion drug parameter setting is performed by inputting the corresponding drug names/abbreviations/colors, etc., so as to facilitate automatic confirmation in the infusion pump 100 or verification by medical care personnel during the infusion process. The volume settings 194-5 allows the user to adjust the alarm volume and/or the volume of other audio output as required. The brightness settings 194-6 allows the user to adjust the screen brightness, alarm light, lighting, and other brightness as required. The network connection settings 195-7 provides an input interface for the user to control the infusion pump 100 and other devices to or not to work in a network connected mode, and a network connected working mode as required. The dock settings 195-8 provides a setting interface for the user to adjust a working parameter of a mounting dock (Dock) connected to the infusion pump 100 as required. The temperature settings 195-9 provides an interface for the user to set a heating temperature of the liquid in the infusion set.

In some embodiments, the foregoing display system includes at least two display screens, at least one of which is formed by stacking a touch layer and a display layer, and the rest may also be formed only by a display layer. Certainly, to achieve a better touch effect for the user, that is, the effect of what you touch is what you get, the display screens included in the display system are all formed by stacking a touch layer and a display layer.

In some embodiments, main components of the foregoing infusion pump are all disposed in a pump body, and the pump door is movably mounted to the pump body and is configured to cover an accommodating cavity for mounting the infusion set. Sometimes when the user opens the pump door, the accommodating cavity for mounting the infusion set can be exposed. The pump door has a front facing the user (the outside) and sides that can be disposed together with a mounting rack, and also has a top and a bottom that can be used to face other stacked infusion pumps. The display system is disposed on the pump door, the display system extends from the left of a midline of the front of the pump door to the right of the midline of the front of the pump door, and a width of the display system is greater than a height of the display system. The display system is disposed substantially as a bar shape on the pump door.

In some embodiments, the width of the display system is greater than or equal to 70% of a width of the front of the pump door, the height of the display system is greater than or equal to 60% of a height of the front of the pump door, or an area of the display system is greater than or equal to 2/3 of an area of the front of the pump door. When a lateral dimension of the pump door is greater than a longitudinal dimension thereof, that is, when the width is greater than the height, the width of the display system is greater than the height thereof, so that a larger display region can be obtained, and the display system presents a rectangle with a horizontal length. The pump door is also provided with physical input keys, which are disposed on one side of the display system. For example, some or all of the physical input keys may be disposed on the right, upper, lower, or left side of the display system. The user can enter data or instructions using the physical input keys, and when the display screen is a touchscreen, the user can also enter data or instructions using the touchscreen. Certainly, the setting of the physical input keys may be used in emergency situations. When the touchscreen fails and infusion control cannot be performed, the user can perform infusion control using the physical input keys, thereby ensuring the use safety of the infusion pump.

During an infusion parameter setting process of an existing infusion pump product, infusion information setting and process confirmation have a random layout and are controlled using physical keys. Before using the infusion pump, medical care personnel need to spend a lot of time learning so as to master rules for the use of the existing infusion pump. Moreover, multiple levels of display interfaces are set, which affects the use by the medical care personnel. However, in the embodiments of the present disclosure, a display system with the integration of a touch layer and a display layer is introduced into the infusion pump. In some embodiments, the use by the medical care personnel in actual working scenarios is further considered, and at least one infusion parameter setting item has a reasonable layout on the display system. In this way, the medical care personnel can properly complete the setting of drug infusion for a patient, which is in line with the user's clinical medication perspective and psychological appeal during use, reduces the cost of learning to the greatest extent based on the application and understanding of an existing clinical medication process, and has an excellent impact on the clinical application process. In particular, after the medical care personnel triggers an image of the infusion parameter setting item, the triggered image of the infusion parameter setting item and an image of a corresponding editing item are displayed on the same screen on the display system. Certainly, depending on procedures of the clinical medication process, images of some other infusion parameter setting items and/or an image of a control item and/or an image of a reading item may further be set, which enables the user to conveniently know information in a same display interface, edit based on the known information, and even control the infusion pump directly on the same display interface. The entire process is very efficient. In addition, displaying the triggered image of the infusion parameter setting item and the image of the corresponding editing item on the same screen on the display system also makes it convenient for the medical care personnel to immediately give error feedback during the editing process, to avoid mistakes.

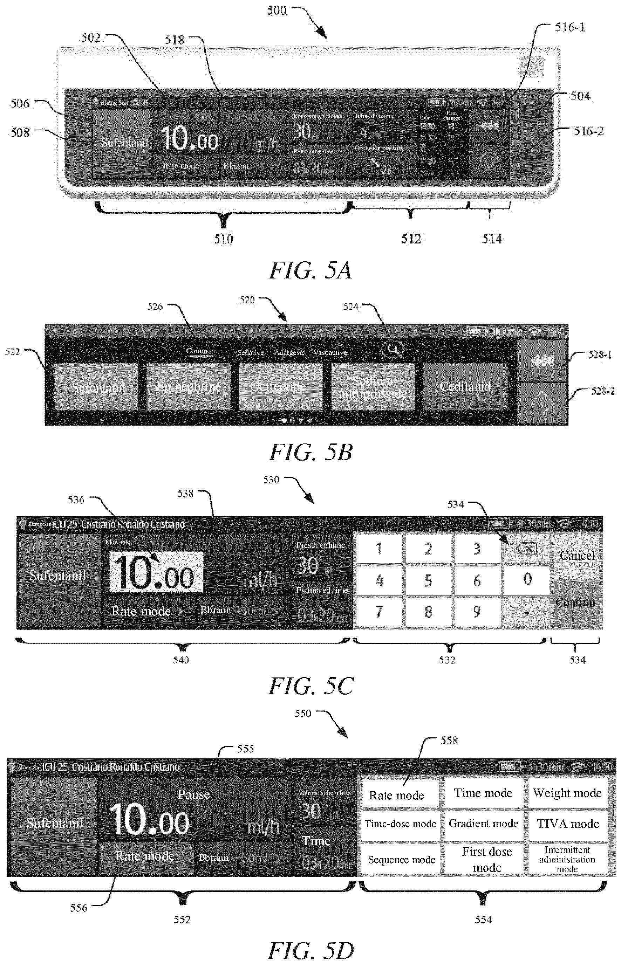

A transfusion pump is taken as an example. The display and settings of a syringe pump are substantially the same as those of the transfusion pump, and details are not described herein again. In some embodiments, as shown in FIGS. 5A to 5F, a processor presents an image including a plurality of infusion parameter setting items 510 in a display interface 502 of a display system of a transfusion pump 500, where the plurality of infusion parameter setting items 510 may include a drug name setting item, a flow rate value setting item, a flow rate unit setting item, an infusion mode setting item, a consumables setting item, a remaining volume setting item, and a remaining time setting item. The display interface 502 further includes an image of a plurality of reading items 512, where the plurality of reading items may include infusion log information, infused volume, and occlusion pressure. The display interface 502 further includes an image of a plurality of control items 514, where the plurality of control items 514 include a bolus control item 516-1 and a pause control item 516-2.

Taking the drug name setting item as an example, in a preset position of the drug name setting item, content of the drug name setting item, that is, a drug name, is displayed, such as "Sufentanil" shown in FIG. 5A. In some cases, the symbol "----" for "To be set" may alternatively be displayed in the preset position of the drug name setting item.

When the processor detects a first trigger event generated based on the drug name setting item, the display system of the transfusion pump 500 presents a display interface 520. As shown in FIG. 5B, the display interface 520 may include an image of a plurality of editing items 522, where the plurality of editing items 522 uses drug names as content, such as "Sufentanil" and "Epinephrine". The editing item image is operable to present content elements, and the content elements may constitute infusion parameter content of the infusion parameter setting item alone or in combination.

The first trigger event generated based on the drug name setting item may be understood as a contact or gesture input by the medical care personnel on the display system, where at least one contact point occurs in a corresponding position of the image of the drug name setting item; according to a preset rule, the processor can identify the first trigger event, and an object triggered by the first trigger event is the drug name setting item. For the principle of a first trigger event generated based on another infusion parameter setting item in the present disclosure, reference may be made to that of the first trigger event generated based on the drug name setting item, and details are not described one by one.

In addition, the display interface 520 further presents auxiliary selection controls 524/526. When the user triggers a position of the control 526 (e.g., the "Common" control), the processor displays a display interface including editing items with the content of names of common drugs on the display screen. When the user triggers a position of the control 524, the processor displays a display interface that may include editing items of content elements of a drug name on the display screen.

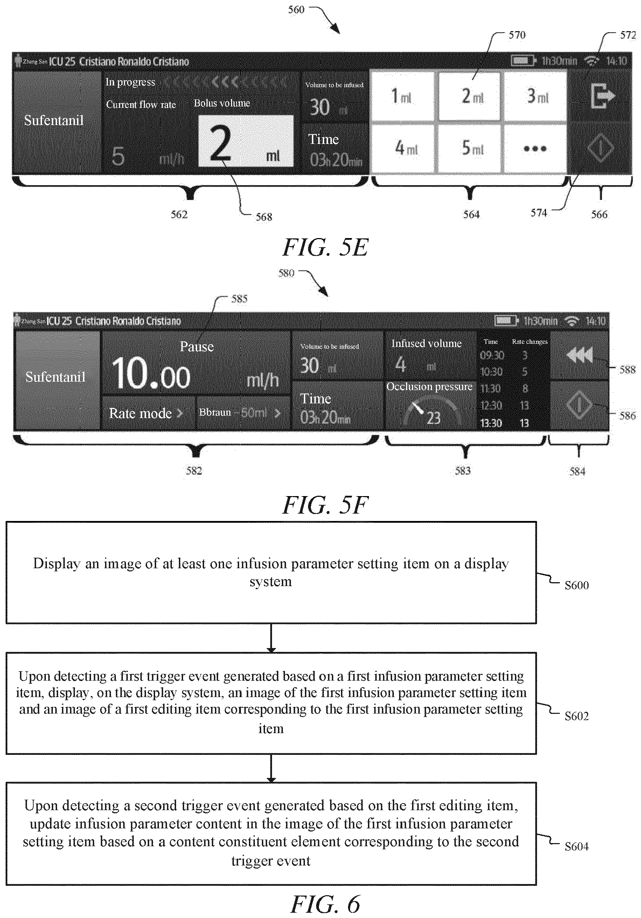

As shown in FIGS. 5F and 5C, when the processor detects a first trigger event generated based on the flow rate value setting item in a display interface 580, the processor displays a display interface 530 on the display system. The display interface 530 includes at least one infusion parameter setting item 540, such as a drug name setting item, a flow rate value setting item, a flow rate unit setting item, an infusion mode setting item, a consumables setting item, a preset volume (remaining volume) setting item, and an estimated time (remaining time) setting item. In addition, an editing item 532 corresponding to the flow rate value is further set at an adjacent position of the infusion parameter setting item 540. Content of the editing item is content elements, and the content elements can constitute the flow rate value alone or in combination.

The display interface 530 further includes at least one control item 534, where the at least one control item 534 includes a cancel control item, a confirm control item, and a backspace control item. When the processor detects that a first trigger event is generated at a corresponding position of a flow rate unit setting item 538, the processor correspondingly displays an image of an infusion parameter setting item 532 including a flow rate value setting item 536 on the display system, and also displays an image of an editing item corresponding to the flow rate unit setting item 538 in the display interface, where the editing item image is operable to present content elements that can constitute a flow rate unit alone or in combination. When detecting a first trigger event generated based on the flow rate value setting item 536 or the flow rate unit setting item 538, the processor presents a highlighted color block image to cover a preset position of the flow rate value setting item 536 or the flow rate unit setting item 538, or presents the infusion parameter content of flow rate value or unit setting visually in aspects such as font, color, font size, or strength and weakness, or generates a sign of an editing pointer at the preset position of the flow rate value setting item or the flow rate unit setting item, to prompt the user that the flow rate value setting item 536 or the flow rate unit setting item 538 is being edited.

As shown in FIGS. 5F and 5D, when the processor detects a first trigger event generated based on the infusion mode setting item in the display interface 580, the processor displays a display interface 550 on the display system. The display interface 550 includes at least one infusion parameter setting item 552, such as a drug name setting item, a flow rate value setting item, a flow rate unit setting item, an infusion mode setting item, a consumables setting item, a volume-to-be-infused (preset volume/remaining volume) setting item, and a remaining time setting item. In addition, an editing item 554 corresponding to the infusion mode setting item is further set at an adjacent position of the infusion parameter setting item 540. The editing item 554 presents content elements that can constitute content of the infusion mode setting item alone. Certainly, the user can also tap a plurality of positions of the editing item 554 multiple times to constitute the infusion mode.

For example, when the processor detects a second trigger event generated based on the editing item 554, and the second trigger event occurs at a preset position of a rate mode 558, the processor uses the rate mode 558 as a content element of the infusion mode setting item, so that infusion parameter content in an infusion mode setting item image 556 is updated to "Rate mode". When detecting a first trigger event generated based on the infusion mode setting item, the processor presents a highlighted color block image to cover a preset position of the infusion mode setting item 556, or presents the infusion parameter content of the infusion mode setting item visually in aspects such as font, color, font size, or strength and weakness, or generates a sign of an editing pointer at the preset position of the infusion mode setting item, to prompt the user that the infusion mode setting item 556 is being edited.

As shown in FIGS. 5A and 5E, when the processor detects a third trigger event generated based on the bolus control item 516 in the display interface 500, the processor displays a display interface 560 on the display system. The display interface 560 includes at least one infusion parameter setting item 562, such as a drug name setting item, a flow rate setting item, a bolus volume setting item 568, a volume-to-be-infused (preset volume/remaining volume) setting item, and a remaining time setting item. In addition, an editing item 564 corresponding to the bolus volume setting item 568 is further set at an adjacent position of the infusion parameter setting item 562. The editing item 564 presents content elements that can constitute content of the bolus volume setting item 568 alone or in combination. The user can tap a plurality of positions of the editing item 564 one or more times to constitute the content of the bolus volume setting item 568. The third trigger event generated based on the bolus control item may be understood as a contact or gesture input by the medical care personnel on the display system, where at least one contact point occurs in a corresponding position of the image of the bolus control item; according to a preset rule, the processor can identify the third trigger event, and an object triggered by the third trigger event is the bolus control item. For the principle of a third trigger event generated based on another control item in the present disclosure, reference may be made to that of the third trigger event generated based on the bolus control item, and details are not described one by one.

For example, when the processor detects that the user triggers a preset position of "2ml" 570 in the editing item 564, the processor uses the content of the bolus volume editing item 564 as a content element of the bolus volume setting item 568, so that content in an image of the bolus volume setting item 568 is updated to "2ml". When detecting the third trigger event generated based on the bolus control item 516-1, the processor presents an image such as a highlighted color block/border at the preset position of the bolus volume setting item 568, to prompt the user that the bolus volume setting item 568 is being edited. In addition, an image such as a highlighted color block/border may also be presented at a trigger position in the editing item 564 in which the second trigger event is generated, to prompt the user that at least one editing item is being triggered.

Moreover, if the infusion parameter setting item 510 in the display interface 500 of FIG. 5A includes a bolus volume setting item, the processor may also detect a first trigger event generated based on the bolus volume setting item, so as to display the display interface 560 on the display system, which enables the user to conveniently set a bolus volume in the display interface 560. In addition, the display interface 560 further includes at least one control item 566, including a control item 572 to return to a previous interface and a control item 574 to start infusion.

As shown in FIGS. 5A and 5E, when the processor detects a third trigger event generated based on the control item 572 to return to the previous interface, the processor displays the display interface 500 on the display system; and when the processor detects a third trigger event based on the control item 574 to start infusion, the processor displays the display interface 500 on the display system, and an infusion progress bar 518 may be displayed in the display interface 500. The infusion progress bar 518 dynamically and visually changes in aspects such as color or length as the infusion work progresses, to prompt the user that the infusion pump 100 is performing the infusion work.

As shown in FIGS. 5A, 5E, and 5F, when the processor detects a third trigger event generated based on the bolus control item 516-1 in the display interface 500, the processor displays the display interface 560 on the display system; and when the processor detects a third trigger event generated based on the pause control item 516-2 in the display interface 500, the processor displays the display interface 580 on the display system. The display interface 580 may include an image of at least one infusion parameter setting item 582, such as a drug name setting item, a flow rate value setting item, a flow rate unit setting item, an infusion mode setting item, a consumables setting item, a volume-to-be-infused setting item, and a remaining time setting item.

In some embodiments, the processor may further display an image of at least one reading item on the display system. As shown in FIG. 5F, the display interface 580 may include an image of at least one reading item 583, such as an infused volume reading item, an occlusion pressure reading item, and an infusion log information reading item. The display interface 580 may further include an image of at least one control item 584, such as a bolus control item 588 and a start infusion control item 586. In some embodiments, the processor may further display prompt information "Pause" in the display interface 580, to prompt the user that the infusion work is paused, and images of at least one infusion parameter setting item, at least one reading item, and at least one control item are set in the display interface, which makes it convenient for the user to quickly set parameters of a next infusion, or to directly start the infusion pump for infusion.

As shown in FIGS. 5A, 5E, and 5F, when the processor detects a third trigger event generated based on the bolus control item 588 in the display interface 580, the processor displays the display interface 560 on the display system, which makes it convenient for the user to set a bolus volume; and when the processor detects a third trigger event generated based on the start control item 586 in the display interface 580, the processor displays the display interface 500 on the display system, and most importantly, the infusion progress bar 518 can be displayed in the display interface 500, to prompt the user that the infusion work is in progress. In addition, images of at least one infusion parameter setting item 510 and/or at least one reading item 512 and/or at least one control item 514 may further be set in the display interface 500.

As shown in FIGS. 5A, 5B, and 5E, when the processor detects a third trigger event generated based on a bolus control item 528-1 in the display interface 520, the processor displays the display interface 560 on the display system, which makes it convenient for the user to set a bolus volume; and when the processor detects a third trigger event generated based on a start infusion control item 528-2 in the display interface 520, the processor displays the display interface 500 on the display system. In some embodiments, the processor may display the infusion progress bar 518 in the display interface 500, to prompt the user that the infusion work is in progress. In addition, images of at least one infusion parameter setting item 510 and/or at least one reading item 512 and/or at least one control item 514 may further be set in the display interface 500.

In the foregoing embodiment, a contact or gesture input that is detected by the processor using the touch layer and the touch module in the display system and that occurs in a region corresponding to the image of the infusion parameter setting item is referred to as a first trigger event. The first trigger event can be generated by a related operation of the contact (which can be implemented using a finger or a stylus, etc.) with the corresponding position of the infusion parameter setting item in the display system. For example, the operation includes determining the presence of the contact at the preset position of the infusion parameter setting item (e.g., detecting a finger-down position), determining a strength of the contact at the preset position of the infusion parameter setting item (such as a force or pressure of the contact), determining that the contact is moving and that at least one contact point is in the preset position of the infusion parameter setting item (e.g., detecting one or more finger drag events), and tracking the movement on the display system and determining that the contact stops or starts at the preset position of the infusion parameter setting item (e.g., detecting a finger-up time or a contact break). The operation of determining the movement of a contact point may include determining a rate (amplitude), a velocity (amplitude and direction), and/or an acceleration (including the amplitude and/or direction) of the contact point. These operations can be applied to a single-point contact or a multi-point contact.

The first trigger event can also be generated by a gesture input at the preset position of the infusion parameter setting item in the display system. For example, detecting a finger tap gesture includes detecting a finger-down event, and then detecting a finger-up event at or near the same position as the finger-down event. For example, detecting a finger swipe gesture includes detecting a finger-down event, then monitoring one or more finger drag events, and then detecting a finger-up event. Similarly, tap, swipe, drag, and other gestures of the stylus are optionally detected by detecting specific contact patterns of the stylus.

In some embodiments, a contact or gesture input that is detected by the processor using the touch layer and the touch module in the display system and that occurs in a preset region of the image of the editing item is referred to as a second trigger event. In some embodiments, a contact or gesture input that is detected by the processor using the touch layer and the touch module and that occurs in a preset region of the image of the control item is referred to as a third trigger event. In some embodiments, a contact or gesture input that is detected by the processor using the touch layer and the touch module and that occurs in a preset region of the display system is referred to as a fourth trigger event. For operations that lead to the second, third, and fourth trigger events, reference may be made to the foregoing example of operations that lead to the first trigger event. The operations leading to the first, second, third, and fourth trigger events may be the same or different. Tactile feedback accompanying the first, second, third, and fourth trigger events may also be the same or different, and the latter is more beneficial to avoiding the user's misoperation.

In some embodiments, FIG. 6 shows an infusion parameter setting method, which can be applied to the infusion pump of the foregoing embodiment. The method includes the following steps.

S600: Display an image of at least one infusion parameter setting item on a display system.

A display interface is displayed on the display system of the infusion pump of the foregoing embodiment, and the display system may include one or more display screens. The image of the infusion parameter setting item may be operable to present infusion parameter content. For example, an image of a drug name setting item may show a drug name that has been set, or a drug name set by default, or a drug name in the previous infusion, or an identifier for representing a drug name to be set, such as "---".

For example, a flow rate value setting item may show a flow rate value that has been set, or a flow rate value set by default, or a flow rate value in the previous infusion, or an identifier for representing a flow rate value to be set, such as "00.0". For example, an image of a flow rate unit setting item may show a flow rate unit that has been set, or a flow rate unit set by default, or a flow rate unit in the previous infusion, or an identifier for representing a flow rate unit to be set. For example, an image of an infusion mode setting item may show an infusion mode that has been set, or an infusion mode set by default, or an infusion mode in the previous infusion, or an identifier for representing an infusion mode to be set. For example, an image of a consumables setting item may show a consumables brand and/or model that have/has been set, or a consumables brand and/or model set by default, or a consumables brand and/or model in the previous infusion, or an identifier for representing consumables to be set. For example, an image of a remaining volume setting item may show a volume to be infused that has been set, or a volume to be infused that is set by default, or a volume to be infused in the previous infusion, or an identifier for representing a volume to be infused that is to be set. For example, an image of a remaining time setting item may show a time to be infused that has been set, or a time to be infused that is set by default, or a time to be infused in the previous infusion, or an identifier for representing a time to be infused that is to be set.

After the infusion pump is powered on, or in some embodiments, after the pump door is closed onto the pump body, the processor may display a display interface including the image of the at least one infusion parameter setting item on the display screen. For example, the display interface may be the display interface shown in FIGS. 5A and 5F.

S602: Display an image of the first infusion parameter setting item and an image of a first editing item corresponding to the first infusion parameter setting item on the display system, when detecting a first trigger event generated based on a first infusion parameter setting item.

The first infusion parameter setting item mentioned here is one of the at least one infusion parameter setting item described above.

The processor displays a display interface that includes both the image of the first infusion parameter setting item and the image of the first editing item corresponding to the first infusion parameter setting item on the display system. The first editing item is also generally one of the at least one editing item, and the image of the editing item is operable to present content elements of the triggered infusion parameter content.

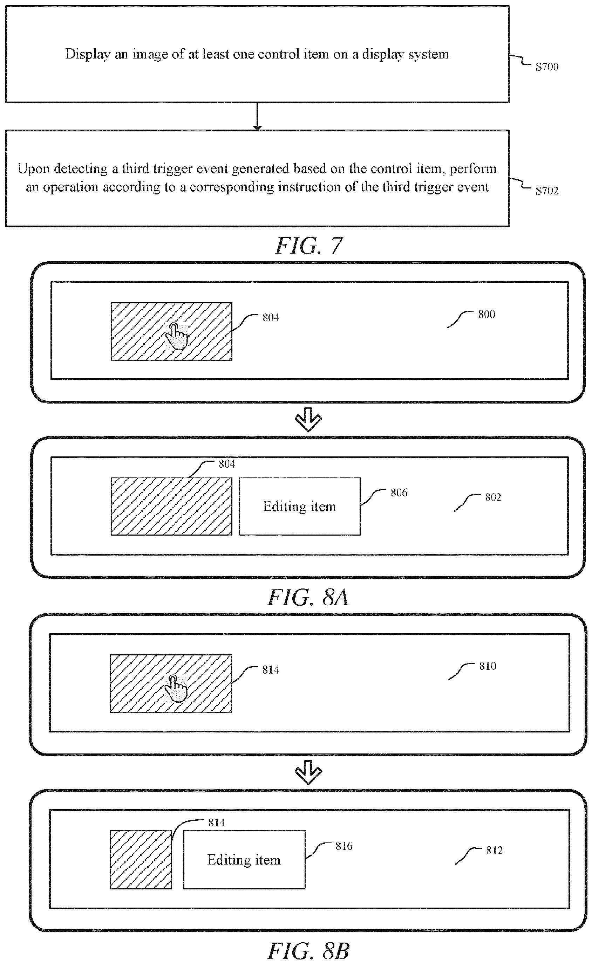

For example, as shown in FIGS. 5F and 5C, when the processor detects that in the display interface 580, an operation such as the user's contact or gesture input occurs at the preset position of the flow rate value setting item, the processor identifies the presence of a first trigger event, and displays the display interface 530 on the display system. The display interface 530 includes the image 536 of the flow rate value setting item and the editing item image 532 corresponding to the flow rate value setting item. The editing item image 532 includes "0" to "9" and ".", a total of 10 characters, at least one of these characters can be used to form a flow rate value, and these characters may be referred to as content elements. Specifically, from another perspective, during the process of the processor jumping from the display interface 580 to the display interface 530, the processor keeps a position and display size of the image of the flow rate value setting item unchanged, displays the corresponding editing item image 532 in a region outside a display position of the flow rate value setting item image, and may add an image of some control items according to actual needs, thereby forming the display interface 530. For the change of the display interface, reference may be made to a display interface 800 and a display interface 802 in FIG. 8A.

In some embodiments, as shown in FIG. 8B, the processor displays a display interface 810 on the display system, and when the processor receives a first trigger event based on a first infusion parameter setting item image 814, the processor reduces a display size of the triggered image 814 of the first infusion parameter setting item, and displays a corresponding first editing item image 816 in a region outside a display position of the triggered image 814 of the first infusion parameter setting item, thereby forming a display interface 812.

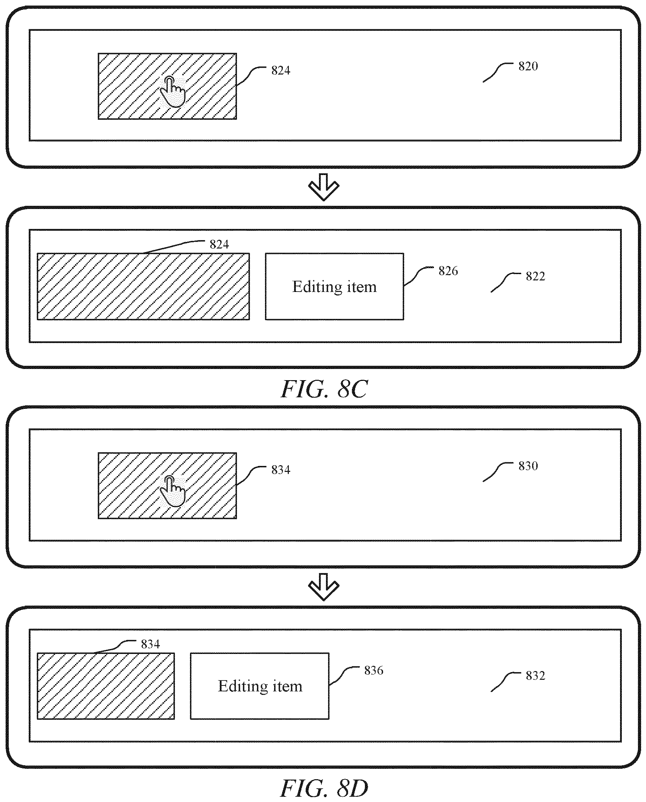

In some embodiments, as shown in FIG. 8C, the processor displays a display interface 820 on the display screen, and when the processor receives a first trigger event based on a first infusion parameter setting item image 824, the processor enlarges a display size of the triggered image 824 of the first infusion parameter setting item, and displays a corresponding first editing item image 826 in a region outside a display position of the triggered image 824 of the first infusion parameter setting item, thereby forming a display interface 822.

In some embodiments, as shown in FIG. 8D, the processor displays a display interface 830 on the display system, and when the processor receives a first trigger event based on a first infusion parameter setting item image 834, the processor moves a display position of the triggered image 834 of the infusion parameter setting item, and displays a corresponding first editing item image 836 in a region outside the display position of the triggered image 834 of the first infusion parameter setting item, thereby forming a display interface 832.

Alternatively, as shown in FIGS. 5F and 5B, when the processor detects that in the display interface 580, an operation such as the user's contact or gesture input occurs at the preset position of the drug name setting item ("Sufentanil"), the processor identifies the presence of a first trigger event, and displays the display interface 520 on the display system. The display interface 520 includes the editing item image 522 corresponding to the drug name setting item, where the editing item image 522 includes a control of a plurality of common drug names such as "Sufentanil", "Epinephrine" "Octreotide", "Sodium nitroprusside", and "Cedilanid". The display interface 520 further includes a drug classification index control. The user triggers the drug classification index control 526 through a contact or gesture input, and a drug name control corresponding to the index control 526 can be presented in the editing item image 522. The display interface 520 further includes a drug retrieval control 524. The user triggers the drug retrieval control 524 through a contact or gesture input, and at least one content element that can form a drug name can be presented in the editing item image 522. The display interface 520, if there is enough space, further includes at least one control item, such as a bolus control item 528-1 and a start infusion control item 528-2.

Certainly, the display interface may further include some other infusion parameter setting items, which may be generally referred to as second infusion parameter setting items, such as a drug name setting item, an infusion mode setting item, and a consumables setting item. This can provide more infusion parameter information for reference by the user when setting the flow rate value, and the user does not need to switch between multiple levels of interfaces. The display interface further includes at least one control item 534, including a delete control item, a cancel control item, and a confirm control item, which makes it convenient for the user to confirm and modify the content entered in the editing item. If the display system has enough space, the display interface may further include a reading item, such as infusion log information, which can be used for reference by the user when setting the flow rate value, without the need for the user to switch between multiple pages. In the display interface 530, the processor further overlays a highlighted color block image for the preset position of the triggered flow rate value setting item, making it convenient for the user to identify a currently operated item. Certainly, information prompt or other visual feedback settings may also be performed in the display interface, for the purpose of making it convenient for the user to identify a currently operated item.

S604: Update infusion parameter content in the image of the first infusion parameter setting item based on a content element corresponding to a second trigger event, when detecting the second trigger event generated based on the first editing item.

Here, the second trigger event generated based on the corresponding position of the editing item image may be understood as that one of the contact points of the operation (the contact or gesture input) leading to the second trigger event occurs in the preset position of one of the editing items to be triggered. For example, the user touches the position of the image of the first editing item; or the user performs a swipe gesture, and a contact start point of the gesture or a contact end point of the gesture is located at the position of the image of the first editing item, or in other words, one of the points of the user's contact or gesture input is associated with the position of the image of the first editing item. The processor detects signal changes at the position of the image of the first editing item in the touch layer, and determines the meaning represented by positions of the signal changes according to a mapping relationship between a position and display content. For example, as shown in FIG. 5C, the processor identifies that a second trigger event is generated in sequence at positions of the content elements "1", "0", ".", "0", and "0", then can identify the content elements "1", "0", ".", "0", and "0" in sequence, and update in sequence in the flow rate value setting item 536, thereby finally constituting the flow rate value "10.00" to be set by the user.

For example, as shown in FIGS. 5B and 5F, the processor identifies that a second trigger event is generated at the position of the content element "Sufentanil", then can display the display interface 580 on the display screen, and updates the drug name content of the image of the drug name setting item in the display interface 580 to "Sufentanil".

In some embodiments, as shown in FIG. 7, the method further includes the following steps.

S700: Display an image of at least one control item on a display system.

As described in the foregoing embodiment, the display interface also includes an image of at least one control item, which may specifically include a bolus control item, a pause control item, a start infusion control item, a cancel control item, a delete control item, a backspace control item, a confirm control item, and a control item to return to a previous interface. These control item images may be operable to present operation content for the infusion pump, and the operation content therefor represents an effect performed by an operation instruction caused by triggering the control item.

S702: Perform an operation according to a corresponding instruction of a third trigger event, when detecting the third trigger event generated based on the control item.

Here, the third trigger event generated based on the control item is understood as that one of the contact points of the operation (the contact or gesture input) leading to the third trigger event occurs in the preset position of one of the control items to be triggered. For example, the user touches the position of the image of the control item; or the user performs a swipe gesture, and a contact start point of the gesture or a contact end point of the gesture is located at the position of the image of the control item, or in other words, one of the points of the user's contact or gesture input is associated with the position of the image of the control item. The processor detects signal changes at the position of the image of the control item in the touch layer, and determines the meaning represented by positions of the signal changes according to a mapping relationship between a position and display content.

The processor detects the third trigger event, and can determine the control item to be triggered according to the associated position of the operation of the third trigger event, and execute according to the operation instruction corresponding to the control item.

For example, as shown in FIG. 5A, the processor displays the display interface 500 on the display system, where the display interface 500 further includes a bolus control item and a pause control item. When the user's contact or gesture input is associated with the corresponding position of the bolus control item image, the processor detects a third trigger event generated based on the bolus control item, and then drives the drive mechanism of the infusion pump according to a preset bolus volume or a set bolus volume, to start a bolus infusion according to the bolus volume. When the user's contact or gesture input is associated with the corresponding position of the pause control item image, the processor detects a third trigger event generated based on the pause control item, and then immediately drives the drive mechanism of the infusion pump to pause the infusion, thereby ensuring the safety of the patient's life in an emergency. In addition, when the user's contact or gesture input is associated with a corresponding position of a power control item image, the processor detects a third trigger event generated based on the corresponding position of the power control item image, and then drives the drive mechanism of the infusion pump to stop the infusion.

Pausing the infusion may be stopping the infusion work but still saving infusion parameters that have been set. Stopping the infusion may be stopping the infusion work, but at least some of the infusion parameters that need to be checked and confirmed by the medical care personnel need to be reset and saved by the medical care personnel.

As shown in FIG. 5B, the processor displays the display interface 520 on the display system, where the display interface 520 further includes a bolus control item and a start infusion control item. The control method and control effect of the bolus control item are as described above. When the user's contact or gesture input is associated with a corresponding position of a start infusion control item image, the processor detects a third trigger event generated based on the start infusion control item, and then drives the drive mechanism of the infusion pump to start the infusion according to the content (drug name) of the infusion parameter setting item that has been set or that is set by default and confirmed by the user.

As shown in FIG. 5C, the processor displays the display interface 530 on the display system, where the display interface 530 includes an editing item image corresponding to the infusion parameter setting item that needs to be set, as well as cancel, confirm, and backspace control items. When the user's contact or gesture input is associated with a corresponding position of a cancel control item image, the processor detects a third trigger event generated based on the cancel control item, and then deletes, in the display interface, an already set content element of the infusion parameter setting item that needs to be set, for example, deletes the flow rate value "10.00". When the user's contact or gesture input is associated with a corresponding position of a confirm control item image, the processor detects a third trigger event generated based on the confirm control item, and then saves the already set content element (e.g., the flow rate value "10.00") of the infusion parameter setting item that needs to be set. When the processor subsequently receives a third trigger event based on the start infusion control item, the infusion can be performed according to the saved infusion parameter (e.g., the flow rate value 10.00). When the user's contact or gesture input is associated with a corresponding position of a backspace control item image, the processor detects a third trigger event generated based on the backspace control item, and then deletes a newly set content element (e.g., the last entered "0"), or deletes the content element before the editing pointer.

As shown in FIG. 5E, the processor displays the display interface 560 on the display system, where the display interface 560 further includes an image of the control item 572 to return to the previous interface. When the user's contact or gesture input is associated with a corresponding position of the image of the control item to return to the previous interface, the processor detects a third trigger event generated based on the control item to return to the previous interface, and then displays the display interface 500 on the display system as shown in FIG. 5A. The content displayed by the display system is changed in the process of switching between the display interface 560 and the display interface 500. For example, the editing item image corresponding to the bolus volume setting item is deleted, and other infusion parameter setting item images may also be adjusted.

In some embodiments, as shown in FIGS. 5C and 5D, the user's contact or gesture input is associated with the corresponding position of the editing item 532; for example, the user enters "1", "0", ".", "0", and "0" in sequence, and the processor also updates with "1", "0", ".", "0", and "0" in sequence at the corresponding position of the flow rate value setting item. In this case, when the processor detects a third trigger event based on the corresponding position of the confirm control item image, the processor may save "10.00" as the flow rate value content for invocation in infusion driving; or when the processor detects a first trigger event based on a corresponding position of another infusion parameter setting item image (for example, the infusion mode setting item), the processor displays the display interface 550, where the display interface 550 stops displaying the editing item corresponding to the flow rate value setting item, but displays the infusion mode setting item and its corresponding editing item, and also saves the content of the flow rate value.

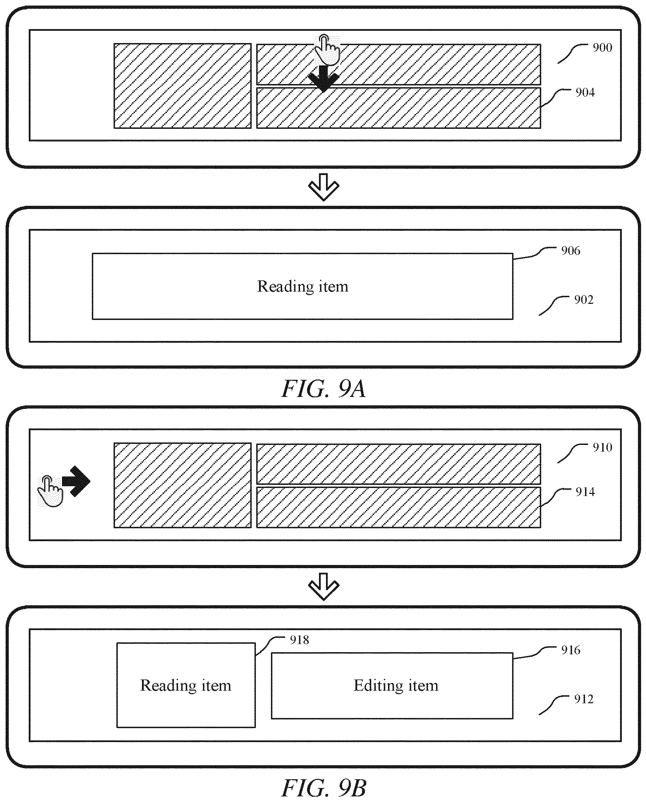

In some embodiments, the processor detects a fourth trigger event. A trigger condition of the fourth trigger event may be identified based on a preset position in the display interface, or may be identified based on other dimensions (e.g., time) of the contact or gesture input, or may be identified based on a position along with other dimensions (e.g., time). As shown in FIG. 9A, when the processor identifies that a fourth trigger event occurs at a preset position of a current display interface (for example, a swipe-down operation from an edge position of the display system), an auxiliary display interface 902 is displayed on the display system. The auxiliary display interface includes a reading item 906, and the reading item 906 may be operable to present at least one of infusion log information, patient information, medical advice information, medication information, integrated device information, infusion device information, or alarm setting information. The reading item 906 may be only for reading, and the user cannot edit through a contact or gesture input.

In some embodiments, as shown in FIG. 9B, when the processor performs a swipe-right operation at a preset position of a display interface 910, such as an edge position of the display interface, the processor identifies the presence of a fourth trigger event, and displays an auxiliary display interface 912 in the display screen. The auxiliary display interface 912 includes a reading item 918, and the reading item 918 may be operable to present at least one of infusion log information, patient information, medical advice information, medication information, integrated device information, infusion device information, or alarm setting information. The read item 918 can be edited or operated through a corresponding editing item 916.

In some embodiments, the reading item may also be set in an infusion parameter setting interface.

The patient information may include patient information obtained from other databases or directly set at the infusion pump, and may include the patient's height, weight, age, gender, admission diagnosis, additional diagnosis, treatment stage, treatment plan, history of allergies, history of diseases, etc. During medication infusion, the patient information is displayed in the current display interface or auxiliary display interface of the infusion pump, which helps the nurse to determine whether the infusion parameters are set correctly, reasonably, and effectively, whether there is any misuse of allergic drugs or drugs that affect other diseases, whether to adjust the rate and dose of medication according to the patient's other diseases, etc.

The medical advice information may include the content, expected execution time, actual execution time, etc. of all the patient's medical advice. During medication infusion, the medical advice information is displayed in the current display interface or auxiliary display interface of the infusion pump, which helps the nurse to grasp the patient's medication as a whole and prepare for the dispensing work; and the doctor or nurse decides whether to adjust the rate and dose of medication according to the execution of the medical advice and the patient's condition.

The medication information may include the patient's medication precautions, medication incompatibility, clinical advice, etc. During medication infusion, the medication information is displayed in the current display interface or auxiliary display interface of the infusion pump, which helps the nurse to determine whether there is any misuse of drugs, whether to adjust the rate and dose of medication, etc.

The integrated device information may include information about a monitor, a ventilator, and other ward instruments, such as vital signs, waveforms, and trends on the monitor, parameters on the ventilator, and medication information on other infusion pumps. During medication infusion, the integrated device information is displayed in the current display interface or auxiliary display interface of the infusion pump, which helps the nurse to synthesize monitoring information of vital signs and medication information of other infusion pumps, and determine whether it is necessary to adjust the rate and dose of medication, etc.

The alarm setting information includes alarm threshold settings related to the current infusion, such as an occlusion pressure alarm threshold, a single air bubble alarm threshold, a cumulative air bubble alarm threshold, and a near completion time. During medication infusion, the alarm setting information is displayed in the current display interface or auxiliary display interface, which helps the nurse to easily view and adjust these settings related to the current infusion alarm, and does not affect the user reading a current infusion status, progress, and other information if displayed in the infusion parameter main interface.

The infusion device information includes other settings related to the current infusion, such as KVO (Keep Vein Open) flow rate, bolus flow rate, and bolus volume limit. Certainly, the infusion device information may further include operating parameters of the current infusion pump device, such as network, time, and operating mode (e.g., day mode or night mode). During medication infusion, the infusion device information is displayed in the current display interface or auxiliary display interface, which helps the medical care personnel to conveniently check, and especially check and adjust these settings related to the current infusion if displayed in the current display interface, which does not affect the medical care personnel reading a current infusion status, progress, and other information.

The infusion log information may include a history of infusion medication for the same patient, specifically including the time of medication and the drug name of the medication and/or other major infusion parameter content, such as a flow rate change at a historical time point, an infusion volume of a drug in a historical unit time period, a cumulative infusion volume at a historical time point, and a cumulative infused volume of all drugs at a historical time point. It is convenient for the medical care personnel to check historical information.

In some embodiments, as shown in FIG. 5A or 5F, the processor displays images of the drug name setting item, the flow rate setting item, and the control item in the display interface 500/580, and the drug name setting item image is set on the left of the display system, and the image of at least one control item is set on the right of the display system. The image of the flow rate setting item may be set alone, or may be set together with an image of another infusion parameter setting item (such as the consumables setting item) between the drug name setting item and the at least one control item. This is in line with operation procedures of the medical care personnel when setting parameters for the infusion pump, and also in line with the habit of the medical care personnel to use the right hand: The drug name is first set, then other infusion parameters (flow rate value, flow rate unit, consumables, and the like.) are set, and then a control operation such as starting the infusion is performed. The overall interface layout is completely in line with the operation procedures in the preparation stage of medication infusion, which is conducive to improving user experience.

Alternatively, the processor arranges the drug name setting item on the left, arranges the rate value and unit setting item on the upper right of the drug name setting item, arranges the infusion mode setting item and the consumables setting item on the right of the drug name setting item and the lower right of the rate setting item, and arranges other infusion parameter setting items on the right of the flow rate value and unit setting items. Generally, the user pays more attention to the drug name and the flow rate than to the infusion mode and consumables. Therefore, the infusion parameter setting items are set from left to right and from top to bottom based on the user's attention, which conforms to the user's usual observation habits and makes the user's checking convenient, and also greatly saves the time of the medical care personnel.

In some embodiments, as shown in FIG. 5A or 5C or 5F, taking the flow rate value setting item as an example, the content in the flow rate value setting item in the display interface 500/530/580, that is, the flow rate value, is separated by ".", a content element of the flow rate value before the "." is presented in a first visual presentation form, and a content element of the flow rate value after the "." is presented in a second visual presentation form. As shown in FIG. 5A or 5C or 5F, the first visual presentation form and the second visual presentation form are different in font size. Similarly, the first visual presentation form and the second visual presentation form may also be different in color (one in red, the other in green), or the first visual presentation form and the second visual presentation form are different in font (one with a large font and the other with a small font), or other manners sufficient to attract visual attention may be used. Alternatively, the first visual presentation form and the second visual presentation form are different in strength and weakness of font. In this way, the medical care personnel can be prompted to pay more attention when setting infusion parameters, which can ensure the safety of infusion and avoid setting errors. Specifically, the flow rate value setting item may be presented in at least two visual presentation forms after the user updates the input content of the editing item, or may be presented in at least two visual presentation forms throughout the entire process.

In some embodiments, taking the flow rate value setting item as an example, when the processor detects a second trigger event based on the editing item corresponding to the flow rate value setting item, the processor updates the flow rate value of the flow rate value setting item in the display interface, and compares whether the updated content exceeds a first limit threshold. If it is determined that the content of the updated flow rate value exceeds the first limit threshold, first prompt information related to the first limit threshold is also output correspondingly in the display interface. The first prompt information may be information such as text or graphics, or at least one change of color, font, or font size is correspondingly made to the updated content.

In some embodiments, taking the flow rate value setting item as an example, when the processor detects a second trigger event generated based on the editing item corresponding to the flow rate value setting item, the processor updates the content of the flow rate value setting item in the display interface, and compares whether the updated content exceeds a second limit threshold. If it is determined that the content of the updated flow rate value exceeds the second limit threshold, second prompt information related to the second limit threshold is also output correspondingly in the display interface. The second prompt information may be information such as text or graphics, or at least one change of color, font, or font size is correspondingly made to the updated content. The first prompt information and the second prompt information are different in a presentation form or presentation content. In some embodiments, if the content of the updated flow rate value exceeds both the second limit threshold and the first limit threshold, the second prompt information corresponding to the second limit threshold is preferentially displayed in the display interface. In some embodiments, if the content of the updated flow rate value exceeds the first limit threshold or the second limit threshold, the processor may also use a tactile feedback mode to perform tactile feedback in the form of sound, image animation, or vibration, so that when editing an editing item, the medical care personnel can perceive whether the updated content exceeds the first or second limit threshold, which is timely and efficient.

The prompt information may include graphics, infusion status display, alarm threshold display, or processing suggestions. Graphics can help the user to see the cause of the alarm at the end of the bed, so that they can be prepared. The infusion status display screen can clearly tell the user whether the patient infusion has stopped, and drive the user to take quick solutions to resume the infusion. The alarm threshold display screen in the user language can clearly tell the user whether the alarm threshold is set reasonably, and assist the user in determining whether the infusion pump is occluded or the infusion pump alarm is caused by the unreasonable setting of the alarm threshold. Handling suggestions enumerate causes of occlusion to help the user to troubleshoot. These are even of more significance when relatively inexperienced new nurses deal with problems.

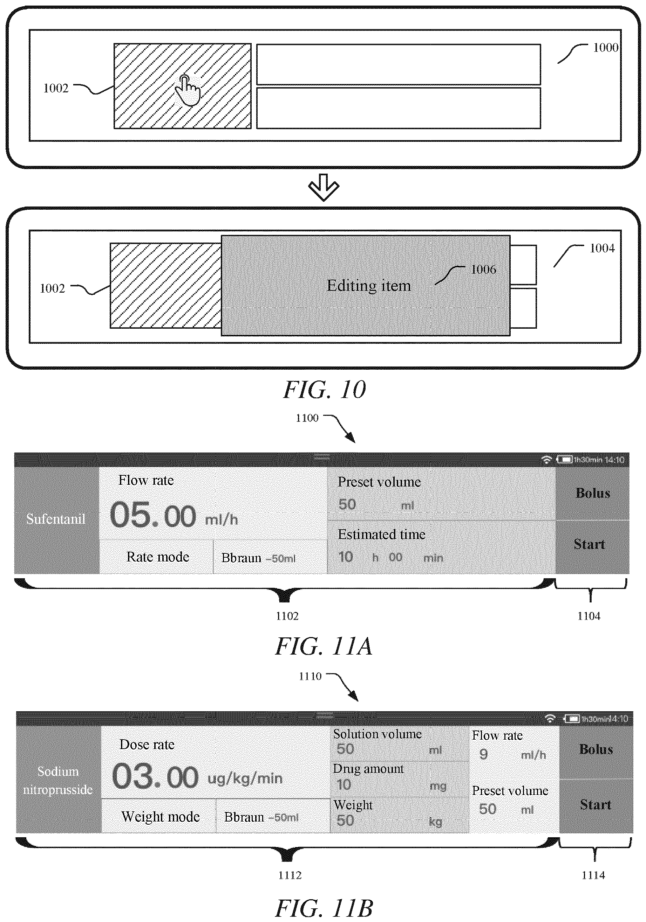

In some embodiments, when the processor detects a first trigger event generated based on a corresponding position of one of the infusion parameter setting items, and displays an image of the triggered infusion parameter setting item and an editing item image corresponding to the triggered infusion parameter setting item on the display system, the displayed editing item image and the image of the triggered infusion parameter setting item may be as shown in FIGS. 5C to 5E, and the two do not overlap. The displayed editing item image and the image of the triggered infusion parameter setting item may also be as shown in FIG. 10. The processor detects a first trigger event based on a preset position of an infusion parameter setting item 1002 in a display interface 1000, and displays a floating window or pop-up window 1008 in a display interface 1000. The display content of the floating window or pop-up window 1008 is an editing item image 1006 corresponding to the triggered infusion parameter setting item 1002, and the triggered infusion parameter setting item image 1002 is also displayed in the display interface, thereby forming a display interface 1004. The floating window can be moved in the display interface by the user through a contact or gesture input. A position of the pop-up window in the display interface is fixed. The floating window or pop-up window of the editing item image 1006 may partially overlap or may not overlap the triggered infusion parameter setting item image 1002, as long as the content display of the triggered infusion parameter setting item image 1002 is not affected.

In some embodiments, as shown in FIGS. 5F, 5D, 11A, and 11B, after the infusion pump is started, the processor first displays a main display interface similar to the display interface 580 on the display system, as shown in FIG. 5F; and when the processor identifies a first trigger event based on the infusion mode setting item, the display interface 550 is displayed, and an infusion mode to be set by the medical care personnel can be determined by identifying a second trigger event of the editing item 554.

For example, in "Rate mode", the display interface 550 is switched to a display interface 1100, and the display interface specifically includes main infusion parameter setting items and control items in the rate mode, which facilitates the setting by the medical care personnel. The display interface 1100 presents parameters that the user needs to set to complete the infusion, including flow the rate value and unit setting items, the consumables setting item, the preset volume setting item, and the estimated time setting item. In this case, the parameters set by the user are the flow rate and the preset volume/time in an order of priority. The flow rate is set first, followed by the preset volume or time, and sometimes the preset volume or time is not set. Therefore, the parameter setting interface is also arranged according to this priority order. Key parameters (parameters that must be set to start the infusion), such as the rate parameter, are placed in a key interactive region, while the preset volume parameter/estimated time parameter, for example, are placed in other regions. The start infusion control item is placed on the right.

For example, in "Weight mode", the display interface 550 is switched to a display interface 1110, and the display interface specifically includes main infusion parameter setting items and control items in the weight mode, which facilitates the setting by the user. The display interface 1110 may include setting items such as a dose-rate setting item, a consumables setting item, concentration (drug amount/solution volume), body weight, flow rate value and unit setting, and a preset volume. In this case, the infusion parameters set by the medical care personnel are the dose-rate, consumables, body weight, concentration (drug amount/solution volume), flow rate, followed by the preset volume in an order of priority, and sometimes the preset volume is not set. Therefore, the display interface 1100 is also arranged according to this priority order. The most critical infusion parameter setting, the dose-rate setting item, is placed in a visual focus region, parameters that must be set to start the infusion, and the weight and concentration (drug amount/solution volume) parameters, are placed in a secondary focus region, while the preset volume parameter, for example, is placed in other regions. The start infusion control item is set on the right of the display system.

In some embodiments, when the processor switches the display interfaces, visual effects presented on the display system may be accompanied. For example, effects such as fade in and fade out, pinwheel, fold, spin, flip, and turn can be used. Specifically, the current display interface is switched to the auxiliary display interface in a fade-in and fade-out manner.

In some embodiments, when the remaining time meets a preset condition, the processor is caused to display prompt information on the display system. During the infusion process, if the user wants to continue to infuse the current drug after the current infusion is completed, the user will pay attention to the remaining time of the current infusion, to determine when to dispense the drug. Especially for some high-alert drugs, if the drug is not dispensed and refilled in time, it may cause fluctuations in the patient's vital signs and affect the development of the patient's condition. In addition, for some drugs with relatively short half-lives, if the drugs are not dispensed and refilled in time, the drug concentration in the patient's blood will also drop rapidly, affecting the efficacy of the drugs and even affecting the vital signs and the patient's condition. Therefore, a prompt of the remaining time at the end of the infusion is particularly important. At the end of the infusion, the processor pops up a graphical prompt on the display system for the remaining time, clearly and exactly prompt the user that the infusion will be completed in a few minutes, and the user can quickly determine the need for dispensing according to the graphical prompt. In addition, how many minutes in advance to be prompted depends completely on the user, and the user can perform personalized configuration in the menu.

In some embodiments, the infusion pump uses a pressure sensor to detect whether a liquid delivery device is occluded, the processor receives an occlusion signal acquired by the pressure sensor, and when the occlusion signal exceeds a first set threshold, occlusion prompt information is output to an input/output system for visual and/or audible prompting. During the infusion process, the processor continuously monitors the operating status of the infusion pump. For example, during the infusion process, the pipeline may be occluded, which affects the infusion of the medicinal solution into the vein. In this case, the user needs to quickly know that an occlusion has occurred, and if the occlusion has affected the infusion, the cause of the occlusion needs to be removed before continuing the infusion. Especially for patients who use high-alert drugs or hypertonic drugs and critically ill patients, stable and uninterrupted infusion is very important and even life-saving. During the infusion process, air bubbles may be generated in the pipeline, and the air bubbles are infused into the vein. In this case, the user needs to quickly know that air bubbles have been generated, and an alarm that the infusion has been affected is issued. The air bubbles in the pipeline need to be removed before continuing the infusion. Especially for children and newborns, a small amount of air bubbles may aggravate the condition of children and even threaten their lives.

In some embodiments, to monitor whether the infusion pump has air bubbles, the transfusion pump uses an air bubble sensor to detect air bubbles in the infusion tube, the processor receives an air bubble signal acquired by the air bubble sensor, and when the air bubble signal exceeds a second set threshold, air bubble prompt information is output to the input/output system for visual and/or audible prompting. Therefore, when an alarm such as occlusions or air bubbles occurs, the graphical prompt and prompt text of the display system can help the user to quickly know the cause for the alarm and the stop of the infusion, and quickly find the occlusion part according to the prompt, find the occlusion cause in turn, and remove the occlusion.

In some embodiments, when the display system includes at least two display screens, if the foregoing infusion parameter setting item, editing item, control item, and/or reading item need to be displayed at the same time, it can be understood that they are simultaneously presented in at least two display screens. For example, the infusion parameter setting item is displayed on one display screen, while the corresponding editing item is displayed on another display screen.

The description has been made with reference to various example embodiments herein. However, persons skilled in the art should appreciate that changes and modifications may be made to the example embodiments without departing from the scope herein. For example, various operation steps and assemblies for executing operation steps may be implemented in different ways according to a specific application or considering any number of cost functions associated with the operation of the system (for example, one or more steps may be deleted, modified, or incorporated into other steps). A transfusion pump is taken as an example for description in some parts herein, and persons skilled in the art should understand that the inventive concept of the embodiments of the present disclosure is also applicable to a syringe pump.

In addition, as understood by persons skilled in the art, the principles herein may be reflected in a computer program product on a computer-readable storage medium that is pre-loaded with computer-readable program codes. Any tangible, non-transitory computer-readable storage medium may be used, including magnetic storage devices (hard disks, floppy disks, and the like), optical storage devices (CD-ROM, DVD, Blu-ray disks, and the like), flash memories, and/or the like. These computer program instructions can be loaded onto a general-purpose computer, a dedicated computer, or other programmable data processing apparatus to form a machine, such that these instructions executed on a computer or other programmable data processing apparatus can generate an apparatus that implements a specified function. These computer program instructions may also be stored in a computer-readable memory that may instruct a computer or other programmable data processing apparatus to operate in a specific manner, such that the instructions stored in the computer-readable memory may form a manufactured product, including an implementation apparatus that implements a specified function. The computer program instructions may also be loaded onto a computer or other programmable data processing apparatus, such that a series of operating steps are executed on the computer or other programmable device to produce a computer-implemented process, such that the instructions executed on the computer or other programmable device can provide steps for implementing a specified function.

Although the principles herein have been shown in various embodiments, many modifications of structures, arrangements, ratios, elements, materials, and components that are particularly suitable for specific environments and operating requirements can be made without departing from the principles and scope of the present disclosure. The above modifications and other changes or amendments will be included within the scope herein.

The above specific description has been described with reference to various embodiments. However, persons skilled in the art would have appreciated that various modifications and changes could have been made without departing from the scope of the present disclosure. Therefore, consideration of the present disclosure will be in an illustrative rather than a restrictive sense, and all such modifications will be included within the scope thereof. Likewise, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "comprise", "include", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communication connection, functional connection, and/or any other connection.

Persons skilled in the art will recognize that many changes may be made to the details of the above-described embodiments without departing from the basic principles of the present disclosure. Therefore, the scope of the present disclosure should be determined according to the claims as follows.

## Claims

1. An infusion parameter setting method, **characterized in that**, the infusion parameter setting method comprises:
displaying an image of at least one infusion parameter setting item on a display system comprising at least one display screen, wherein the infusion parameter setting item image is operable to present infusion parameter content, and the at least one infusion parameter setting item comprises a first infusion parameter setting item;
displaying the image of the first infusion parameter setting item and an image of a first editing item corresponding to the first infusion parameter setting item on the display system, when detecting a first trigger event generated based on the first infusion parameter setting item, wherein the first editing item image is operable to present a content element of the first infusion parameter setting item; and
updating the infusion parameter content in the image of the first infusion parameter setting item, according to the content element corresponding to a second trigger event, when detecting the second trigger event generated based on the first editing item.

2. The method according to claim 1, **characterized in that**, the method further comprises:
displaying an image of at least one control item on the display system, wherein the control item image is operable to present operation content for an infusion pump;
performing at least one of the following operations according to a corresponding instruction of a third trigger event, when detecting the third trigger event generated based on the control item:
saving infusion parameter content of the first infusion parameter setting item;
deleting at least part of infusion parameter content of the first infusion parameter setting item;
driving a drive mechanism of the infusion pump to start infusion based on saved infusion parameter content of the first infusion parameter setting item;
driving a drive mechanism of the infusion pump to stop or pause infusion; and
at least partly changing the image of the infusion parameter setting item or the image of the editing item that is displayed on the display system.

3. The method according to claim 1, **characterized in that**, the at least one infusion parameter setting item further comprises a second infusion parameter setting item, and after updating the infusion parameter content in the image of the first infusion parameter setting item, the method further comprises:
saving the updated infusion parameter content of the first infusion parameter setting item and stopping displaying the image of the first editing item, and displaying an image of the second infusion parameter setting item and an image of a second editing item corresponding to the second infusion parameter setting item on the display system, when detecting a first trigger event generated based on the second infusion parameter setting item.

4. The method according to claim 1, 2, or 3, **characterized in that**, after detecting a first trigger event generated based on a corresponding position of the first infusion parameter setting item, the method further comprises:
displaying a visual feedback image based on the first infusion parameter setting item on the display system.

5. The method according to claim 1, 2, or 3, **characterized in that**, displaying the image of the first infusion parameter setting item and an image of a first editing item corresponding to the first infusion parameter setting item on the display system, comprises:
moving a display position of the image of the first infusion parameter setting item, and displaying the corresponding image of the first editing item in a region outside the display position of the image of the first infusion parameter setting item; or
reducing a display size of the image of the first infusion parameter setting item, and displaying the corresponding image of the first editing item in a region outside a display position of the image of the first infusion parameter setting item; or
keeping display position and size of the image of the first infusion parameter setting item unchanged, and displaying the corresponding image of the first editing item in a region outside a display position of the image of the first infusion parameter setting item; or
enlarging a display size of the image of the first infusion parameter setting item, and displaying the corresponding image of the first editing item in a region outside a display position of the image of the first infusion parameter setting item.

6. The method according to claim 1, **characterized in that**, the method further comprises:
switching a current display interface of an infusion pump to an auxiliary display interface corresponding to a fourth trigger event, when detecting the fourth trigger event, wherein the auxiliary display interface is configured to present infusion log information, patient information, medical advice information, medication information, integrated device information, infusion device information, or alarm setting information.

7. The method according to claim 2, **characterized in that**, the at least one infusion parameter setting item comprises a drug name setting item and a flow rate setting item, and displaying an image of at least one infusion parameter setting item and an image of at least one control item in a display interface comprises:
displaying images of the drug name setting item, the flow rate setting item, and the at least one control item on the display system, wherein the image of the drug name setting item is set on the left of the display system, the image of the at least one control item is set on the right of the display system, and the image of the flow rate setting item is set adjacent to the image of the drug name setting item.

8. The method according to claim 1, 2, or 3, **characterized in that**, the method further comprises:
displaying an image of at least one reading item on the display system, wherein the reading item image is operable to present infusion log information or the infusion device information.

9. The method according to claim 1, 2, or 3, **characterized in that**, after updating the infusion parameter content in the image of the first infusion parameter setting item, the method comprises:
displaying the updated infusion parameter content of the first infusion parameter in at least two visual presentation forms.

10. The method according to claim 1, 2, or 3, **characterized in that**, the first infusion parameter setting item is a flow rate value setting item; and after updating the infusion parameter content in the image of the first infusion parameter setting item, the method further comprises:
correspondingly outputting first prompt information related to a first limit threshold or second prompt information related to a second limit threshold on the display system, when determining that the updated content of the first infusion parameter setting item exceeds the first limit threshold or the second limit threshold.

11. An infusion pump, **characterized in that**, the infusion pump is used together with an infusion set, and the infusion set is operable to deliver a fluid substance in the infusion set into a patient's body; and the infusion pump comprises:
a drive apparatus, which is operable to apply pressure to the infusion set, such that the fluid substance in the infusion set moves directionally;
an input/output system, which is operable to provide an interface between an input/output peripheral and a peripheral device interface;
the input/output peripheral, which comprises a display system for providing a visual display interface, wherein the display system comprises at least one display; and
a processor, configured to display an image of at least one infusion parameter setting item on the display system, wherein the infusion parameter setting item image is operable to present infusion parameter content, and the at least one infusion parameter setting item comprises a first infusion parameter setting item; and further configured to, display the image of the first infusion parameter setting item and an image of a first editing item corresponding to the first infusion parameter setting item on the display system, when detecting a first trigger event generated based on the first infusion parameter setting item, wherein the first editing item image is operable to present a content element of the first infusion parameter setting item; and update the infusion parameter content in the image of the first infusion parameter setting item based on a content element corresponding to a second trigger event, when detecting the second trigger event generated based on the first editing item.

12. The infusion pump according to claim 11, **characterized in that**, the processor is further configured to: display an image of at least one control item on the display system, wherein the control item image is operable to present an operation instruction for the infusion pump; and when detecting a third trigger event generated based on the control item, save infusion parameter content of the first infusion parameter setting item according to a corresponding instruction of the third trigger event.

13. The infusion pump according to claim 11, **characterized in that**, the processor is further configured to: display an image of at least one control item on the display system, wherein the control item image is operable to present an operation instruction for the infusion pump; and when detecting a third trigger event generated based on the control item, delete at least part of infusion parameter content of the first infusion parameter setting item according to a corresponding instruction of the third trigger event.

14. The infusion pump according to claim 11, **characterized in that**, the processor is further configured to: display an image of at least one control item on the display system, wherein the control item image is operable to present an operation instruction for the infusion pump; and when detecting a third trigger event generated based on the control item, drive a drive mechanism of the infusion pump to start infusion based on saved infusion parameter content of the infusion parameter setting item, according to a corresponding instruction of the third trigger event.

15. The infusion pump according to claim 11, **characterized in that**, the processor is further configured to: display an image of at least one control item on the display system, wherein the control item image is operable to present an operation instruction for the infusion pump; and when detecting a third trigger event generated based on the control item, drive a drive mechanism of the infusion pump to stop or pause infusion according to a corresponding instruction of the third trigger event.

16. The infusion pump according to claim 11, **characterized in that**, the processor is further configured to: display an image of at least one control item on the display system, wherein the control item image is operable to present an operation instruction for the infusion pump; and when detecting a third trigger event generated based on the control item, at least partly change the image of the infusion parameter setting item or the image of the editing item that is displayed on the display system according to a corresponding instruction of the third trigger event.

17. The infusion pump according to claim 11, **characterized in that**, the at least one infusion parameter setting item further comprises a second infusion parameter setting item, and the processor is further configured to: after updating the infusion parameter content in the image of the first infusion parameter setting item, save the updated content of the first infusion parameter setting item and stop displaying the image of the first editing item, and display an image of the second infusion parameter setting item and an image of a second editing item corresponding to the second infusion parameter setting item on the display system, when detecting a first trigger event generated based on the second infusion parameter setting item.

18. The infusion pump according to any one of claims 11 to 13, **characterized in that**, the processor is further configured to: after detecting a first trigger event generated based on the first infusion parameter setting item, display a visual feedback image based on the first infusion parameter setting item on the display system.

19. The infusion pump according to any one of claims 11 to 13, **characterized in that**, the processor is further configured to move a display position of the image of the first infusion parameter setting item, and display the corresponding image of the first editing item in a region outside the display position of the image of the first infusion parameter setting item, so as to display the image of the first infusion parameter setting item and the image of the first editing item corresponding to the first infusion parameter setting item on the display system; or
the processor is further configured to reduce a display size of the image of the first infusion parameter setting item, and display the corresponding image of the first editing item in a region outside a display position of the image of the first infusion parameter setting item, so as to display the image of the first infusion parameter setting item and the image of the first editing item corresponding to the first infusion parameter setting item on the display system; or
the processor is further configured to keep display position and size of the image of the first infusion parameter setting item unchanged, and display the corresponding image of the first editing item in a region outside a display position of the image of the first infusion parameter setting item, so as to display the image of the first infusion parameter setting item and the image of the first editing item corresponding to the first infusion parameter setting item on the display system; or
the processor is further configured to enlarge a display size of the image of the first infusion parameter setting item, and display the corresponding image of the first editing item in a region outside a display position of the image of the first infusion parameter setting item, so as to display the image of the first infusion parameter setting item and the image of the first editing item corresponding to the first infusion parameter setting item on the display system.

20. The infusion pump according to claim 11, **characterized in that**, the processor is further configured to switch a current display interface of the infusion pump to an auxiliary display interface corresponding to a fourth trigger event, when detecting the fourth trigger event, wherein the auxiliary display interface is configured to present infusion log information, patient information, medical advice information, medication information, integrated device information, infusion device information, or alarm setting information.

21. The infusion pump according to any one of claims 12 to 16, **characterized in that**, the at least one infusion parameter setting item comprises a drug name setting item and a flow rate setting item, wherein an image of the drug name setting item is set on the left of the display system, an image of the at least one control item is set on the right of the display system, and the image of the flow rate setting item is set adjacent to the image of the drug name setting item.

22. The infusion pump according to any one of claims 11 to 17, **characterized in that**, an image of at least one reading item is further displayed on the display system, wherein the image of the reading item is operable to present infusion log information or the infusion device information.

23. The infusion pump according to any one of claims 11 to 17, **characterized in that**, the updated infusion parameter content of the first infusion parameter is displayed on the display system in at least two visual presentation forms.

24. The infusion pump according to any one of claims 11 to 17, **characterized in that**, the first infusion parameter setting item is a flow rate value setting item; and the processor is further configured to, after updating the infusion parameter content in the image of the first infusion parameter setting item, correspondingly output first prompt information related to a first limit threshold or second prompt information related to a second limit threshold on the display system, when determining that the updated content of the first infusion parameter setting item exceeds the first limit threshold or the second limit threshold.

25. An infusion pump, **characterized in that**, the infusion pump is used together with an infusion set, and the infusion set is operable to deliver a fluid substance in the infusion set into a patient's body; and the infusion pump comprises:
a pump body;
a drive apparatus, which is operable to apply pressure to the infusion set, such that the fluid substance in the infusion set moves directionally;
a pump door, which is movably mounted to the pump body and configured to cover an accommodating cavity for mounting the infusion set, or configured to expose the accommodating cavity for mounting the infusion set, wherein the pump door has a front that faces outside;
an input/output system, which is operable to provide an interface between an input/output peripheral and a peripheral device interface;
the input/output peripheral, which comprises a display system for providing a visual display interface, wherein the display system comprises at least one display; and the display system is disposed on the pump door, the display system extends from the left of a midline of the front of the pump door to the right of the midline of the front of the pump door, and a width of the display system is greater than a height of the display system; and
a processor, configured to, display an image of at least one infusion parameter setting item on the display system, after the pump door is closed onto the pump body, wherein the infusion parameter setting item image is operable to present infusion parameter content, and the at least one infusion parameter setting item comprises a first infusion parameter setting item; and further configured to display the image of the first infusion parameter setting item and an image of a first editing item corresponding to the first infusion parameter setting item on the display system, when detecting a first trigger event generated based on the first infusion parameter setting item, wherein the first editing item image is operable to present a content element of the first infusion parameter setting item; and update the infusion parameter content in the image of the first infusion parameter setting item based on a content element corresponding to a second trigger event, when detecting the second trigger event generated based on the first editing item.
